(19) Europäisches Patentamt
European Patent Office
Office européen des brevets

(11) Publication number: **0 163 238 B1**

# (12) EUROPEAN PATENT SPECIFICATION

(45) Date of publication of patent specification: **24.07.91**

(51) Int. Cl.5: **C07D 495/08**, C07D 493/08, C07D 211/90, A61K 31/44

(21) Application number: **85106202.6**

(22) Date of filing: **21.05.85**

The file contains technical information submitted after the application was filed and not included in this specification

## (54) Substituted and bridged pyridines useful as pharmaceutical agents.

(30) Priority: **29.05.84 US 614885**

(43) Date of publication of application:
**04.12.85 Bulletin 85/49**

(45) Publication of the grant of the patent:
**24.07.91 Bulletin 91/30**

(84) Designated Contracting States:
**CH DE FR GB IT LI NL**

(56) References cited:
**EP-A- 0 088 274**
**EP-A- 0 102 046**
**US-A- 4 132 710**
**US-A- 4 305 938**

**PHARMAZIE HEUTE, vol. 104, no. 3, 1983, pp. 139-146**

**HOUBEN-WEYL, Vol. E 11, "Organische Schwefel-Verbindungen", Ed. D. Klamann, pp. 186-187 & pp. 872-873**

**JOURNAL OF MEDICINAL CHEMISTRY, vol. 30, 1987, pp. 690-695**

(73) Proprietor: **MERCK & CO. INC.**
**126, East Lincoln Avenue P.O. Box 2000**
**Rahway New Jersey 07065-0900(US)**

(72) Inventor: **Claremon, David A.**
**809 Mockingbird Lane**
**Audubon, PA 19403(US)**

(74) Representative: **Abitz, Walter, Dr.-Ing. et al**
**Abitz, Morf, Gritschneder, Freiherr von Wittgenstein Postfach 86 01 09**
**W-8000 München 86(DE)**

EP 0 163 238 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

Substituted dihydropyridines are known to be useful for reducing blood pressure, effecting dilation of the coronary vessels, and preventing vasospasms. Typical of such substituted dihydropyridines are those disclosed in U.S. Patents 3,923,818; 3,905,970; 4,044,141; 4,237,137; and, 4,285,955. The substituted dihydropyridines disclosed in these patents do not include bridged ring structures.

Weller et al. [J. Org. Chem, 48, 3061-67 (1983)] disclose fused ring and bridged ring structures (e.g., spiro piperidines, spiro pyridines) but indicate that in such structures no exceptionally active analgesics have been found. Furthermore, the bridge portion in these structures is from the ortho position of the benzene ring to the para position of the piperidine or pyridine rings.

4-Phenyl-1,4-dihydropyridines without bridged ring structures are known from EP-A-0088274 and Pharmazic heute 104(3), 139-146 (1983). 3-Phenyl-1,2,5,6-tetrahydropyridines, 3-Phenyl-1,2,3,4-tetrahydropyridines and 3- or 4-Phenylpiperidines-all containing a bridge between the phenyl group and the tetrahydropyridine group or the piperidine group - are known from U.S. Patents 4,132,710 and 4,305,938.

### SUMMARY OF THE INVENTION

This invention relates to novel substituted and bridged pyridines and related compounds which are useful as calcium channel blockers and to methods for preparing such compounds. The compounds of this invention can be represented by the general formula:

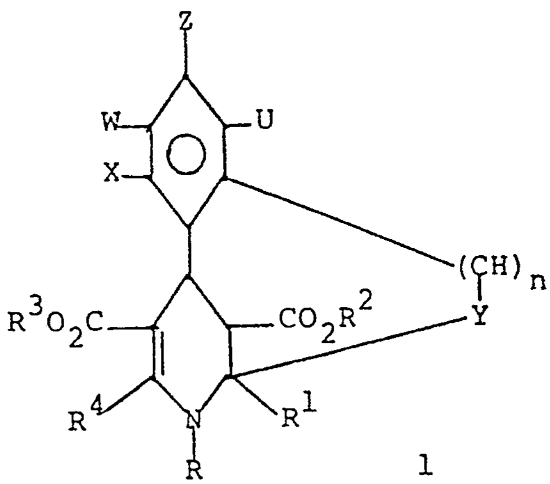

1

wherein:
R is hydrogen;
$C_1$-$C_8$ straight chain or branched alkyl;
benzyl;
$R^1$ and $R^4$ are independently hydrogen;
straight chain or branched, saturated hydrocarbon having up to 8 carbon atoms;
hydroxy-$C_1$-$C_8$-alkyl;
$CO_2$-loweralkyl of $C_1$-$C_8$
$C_3$-$C_8$ cycloalkyl;
$R^2$ and $R^3$ are independently straight chain or branched, saturated or unsaturated hydrocarbon having up to 8 carbon atoms;
$C_3$-$C_8$ cycloalkyl;
$C_2$-$C_8$ alkyl interrupted by 1 to 2 oxygen atoms or substituted with $NR^5R^6$ wherein $R^5$ and $R^6$ can independently be hydrogen, $C_1$-$C_8$ alkyl, or aralkyl wherein the alkyl group has up to 8 carbon atoms and the aryl has 6 carbon atoms,
or $R^5$ and $R^6$ together with the N atom can be joined to form a 5- or 6-membered ring wherein the 6-membered ring can contain an O, S, or N heteroatom and the N heteroatom can be substituted with $C_1$-$C_6$alkyl;

X, W, Z and U can independently be hydrogen;
phenyl and substituted phenyl wherein the substituents can be 1-2 halo (Cl, Br, F) atoms;
halo;
$NO_2$;
trifluoromethyl;
$C_1$-$C_8$alkoxy;
$C_1$-$C_8$ linear or branched alkyl, linear or branched alkenyl or alkynyl having up to 8 carbon atoms;
$C_1$-$C_8$ thioalkyl wherein the S atom can be substituted with 1-2 O atoms;
cyano;
$NH_2$;

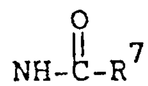

wherein $R^7$ is $C_{1-8}$ alkoxy or $C_1$-$C_8$ alkyl; provided that two of X, W, Z and U are hydrogen;
or X and W, W and Z, or Z and U, together with the phenyl ring, form naphthyl or benzooxadiazole rings;
Y is O; S; SO;
n is 0 or 1;
and, the pharmaceutically acceptable acid addition salts thereof; with the proviso that X cannot be $C_1$-$C_8$ alkoxy or $C_2$-$C_8$ alkenyl.

Preferred are compounds of Formula 1 wherein:
R is hydrogen;
$R^1$ and $R^4$ are independently $C_{1-8}$ alkyl;
$R^2$ and $R^3$ are independently $C_{1-8}$ alkyl which can be substituted with $NR^5R^6$ wherein $R^5$ and $R^6$ are as defined above;
X is hydrogen or halo;
U and W can each be $NO_2$; $CF_3$; or halo;
Z is hydrogen;
Y is O or S; and,
n is 0 or 1.

Most preferred are those compounds of Formula 1 wherein:
R is hydrogen;
$R^1$ and $R^4$ are methyl;
$R^2$ and $R^3$ are $C_{1-4}$ alkyl;
X is hydrogen or halo;
U and W can each be $NO_2$, $CF_3$ or halo;
Z is hydrogen;
Y is O or S; and,
n is 0 or 1.

The invention further relates to compounds having the formula:

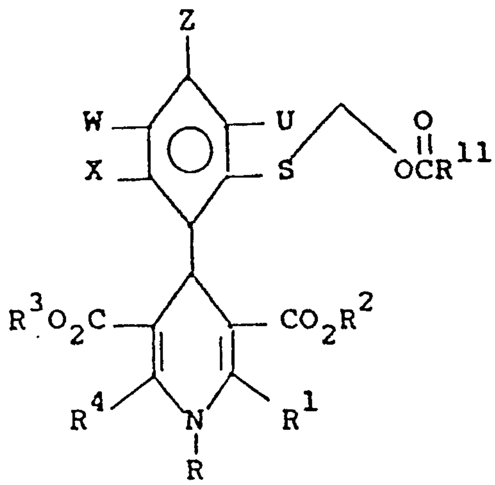

14

wherein R-$R^4$, X, W, Z and U are as defined above and, $R^{11}$ is $C_1$-$C_8$ loweralkyl.

The compounds of the invention can be prepared by the methods set forth hereinbelow. In these methods, unless otherwise indicated, the starting materials employed are commercially available or known in the literature, R-$R^4$, W, X, Y, Z, U, and n are as defined above, and all temperatures are in degrees Celsius.

REACTION SCHEME I

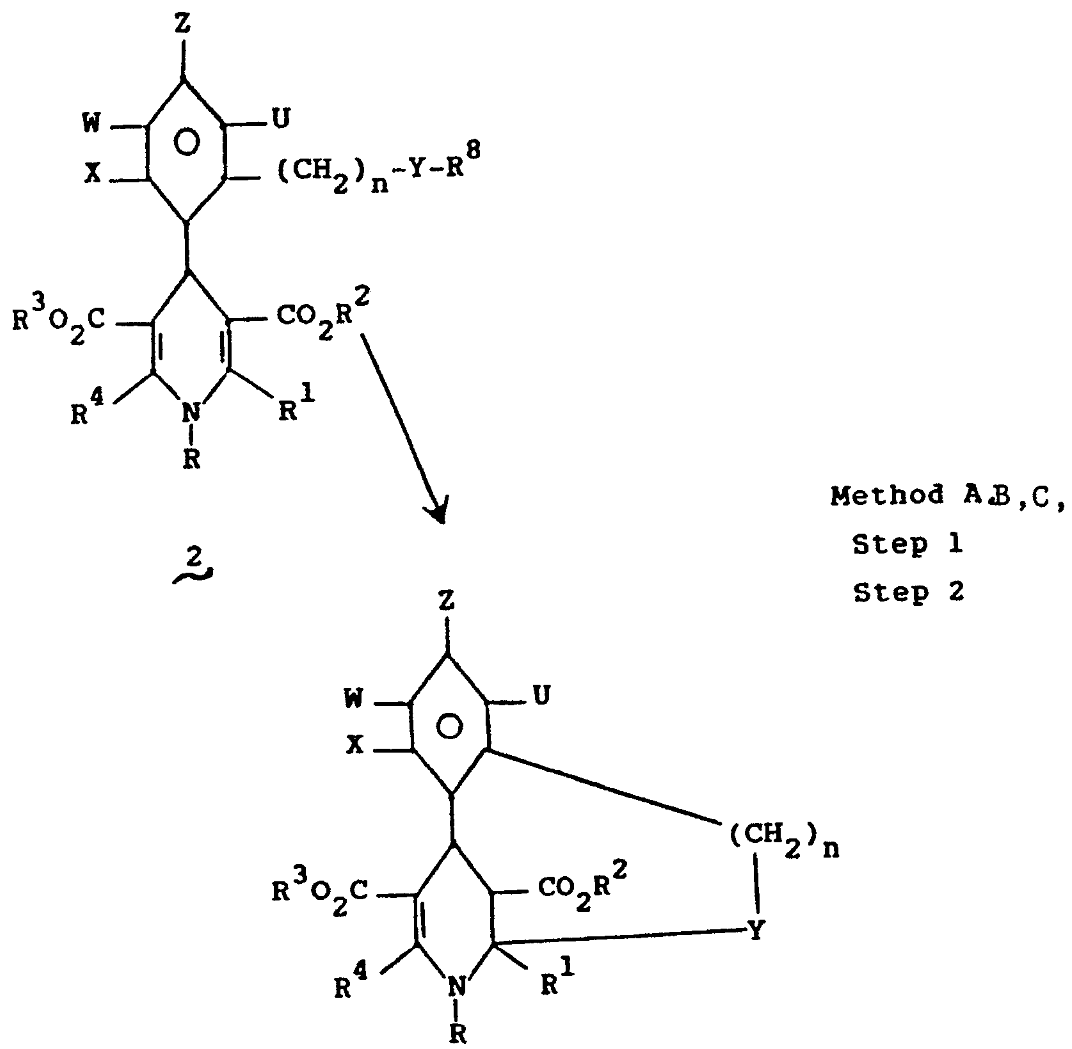


2

1a
(Y=O; n=O)

METHOD A

Step 1: As shown in Reaction Scheme I, either dihydropyridine

2

($R^8 = C_1$-$C_8$ alkyl, benzyl, paramethoxy benzyl; X, W, Z and U are not alkoxy, Y is O, and n is ∅) or W and X, W and Z, or Z and U with the phenyl form napthyl [both of which compounds can be prepared as described in U.S. Patent 3,959,296] is treated with about 1-5 mole equivalent (meq) (preferably 1 meq) of a boron halide or boron halide complex such as boron tribromide, ($BBr_3$), boron trichloride, ($BCl_3$), boron tribromide/dimethylsulfide ($BBr_3$/DMS), dimethylboron bromide or pyridine/hydrochloride or aluminum chloride ($AlCl_3$) (preferably $BBr_3$) neat or in a suitable solvent such as methylene chloride ($CH_2Cl_2$), chloroform ($CHCl_3$), benzene (preferably ($CH_2Cl_2$)) at a temperature of about -78° to the reflux temperature of the solvent (preferably 25° C to reflux) under an inert atmosphere (Ar or $N_2$) for a period of about 1-25 hours

(preferably 1 hour). The intermediate lactone compound

3

(Reaction Scheme II, Method D) is isolated and treated as described below in Step 2.

Step 2: The reaction mixture, maintained under the inert atmosphere, is then treated with about 2-5 meq (preferably 5 meq) potassium carbonate, potassium hydride, lithium hydride, or sodium hydride (preferably potassium carbonate) and 2-25 meq of $R^2OH$, preferably about 10 meq, neat or in a suitable solvent such as dimethylformamide (DMF), dimethoxyethane (DME) or tetrahydrofuran (THF) at about 25°-125°C (preferably about 25°C) for about 1-48 hours to afford a compound

1a

of Formula 1.

Method B

Step 1: In this method ($R^8 = CH_2\text{-}C_6H_5$, Y = O, n = 0, 1), compound

2

(Reaction Scheme I) is treated with hydrogen under pressure (1 atm. or 2-3 atm.) in the presence of about 10%-100% weight equivalents of a catalyst such as 5% Pd/C, 10% Pd/C or $PtO_2$ in a suitable solvent such as ethanol (EtOH), ethyl acetate, methanol ($CH_3OH$), isopropanol, petroleum ether, preferably EtOH, at 10°-40°C (preferably 25°C) for about 25-36 hours to obtain compound

1a.

Step 2: Compound

2

is first treated as described above in Method B, Step 1, to obtain an intermediate phenol dihydropyridine which is then treated with about 0.1-0.3 meq (preferably 0.1 meq) of an acid (toluenesulfonic acid, camphorsulfonic acid, trifluoroacetic acid, methanesulfonic acid, sulfuric acid; preferably, camphorsulfonic acid) in a solvent such as $CH_2Cl_2$, $CHCl_3$, dichloroethane, THF, DME, or mixtures thereof (preferably methylene chloride) at about 25 to 50°C for about 2-24 hours to obtain compound

1a.

Method C

Dihydropyridine

2

($R^8 = CH_2\text{-}C_6H_5$ or

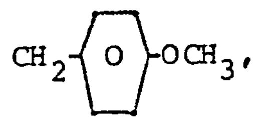

Y = O or S, n = 0 or 1) (Reaction Scheme I) is treated under an inert atmosphere (Ar or $N_2$) with about 5-50 meq (preferably 10 meq) trifluoroacetic acid neat or in the presence of a solvent (anisole, $CH_2Cl_2$, $CHCl_3$, dichloroethane) at 25°-100°C (preferably 25°C) for about 10-60 minutes to obtain the Formula 1 compound.

## REACTION SCHEME II, Method D

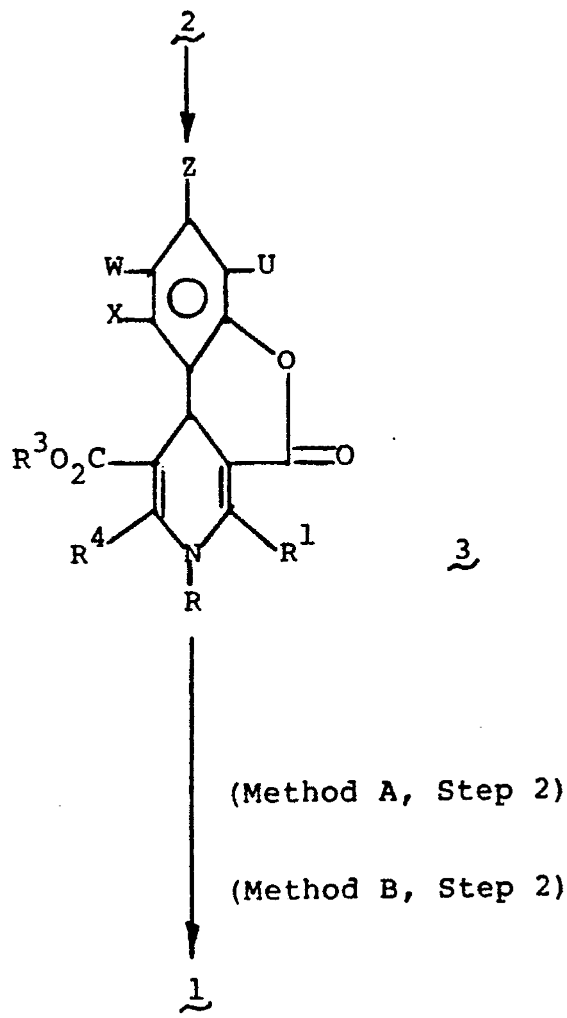


2

3

1

Method D

In this method (Reaction Scheme II $R^8 = C_1$-$C_8$alkyl, benzyl, paramethoxybenzyl; Y = O; n = O; and, X, W, Z and U are not alkoxy), dihydropyridine or naphthyldihydropyridine

2

is treated under an inert atmosphere (Ar or $N_2$) with about 1 meq $BBr_3$ or $BCl_3$ in an organic solvent ($CH_2Cl_2$, dichloromethane, $CHCl_3$, benzene; preferably $CH_2Cl_2$) at a temperature of about -78° to 40°C for about 3 hours to obtain oxo-carbonyl bridged dihydropyridine intermediate

3.

This intermediate

3,

is then isolated and treated as described above in Method A, Step 2, to obtain the phenol dihydropyridine intermediate (Method B, Step 2) which is treated as described above in Method B, Step 2, to afford compound

1a.

REACTION SCHEME III, METHOD E

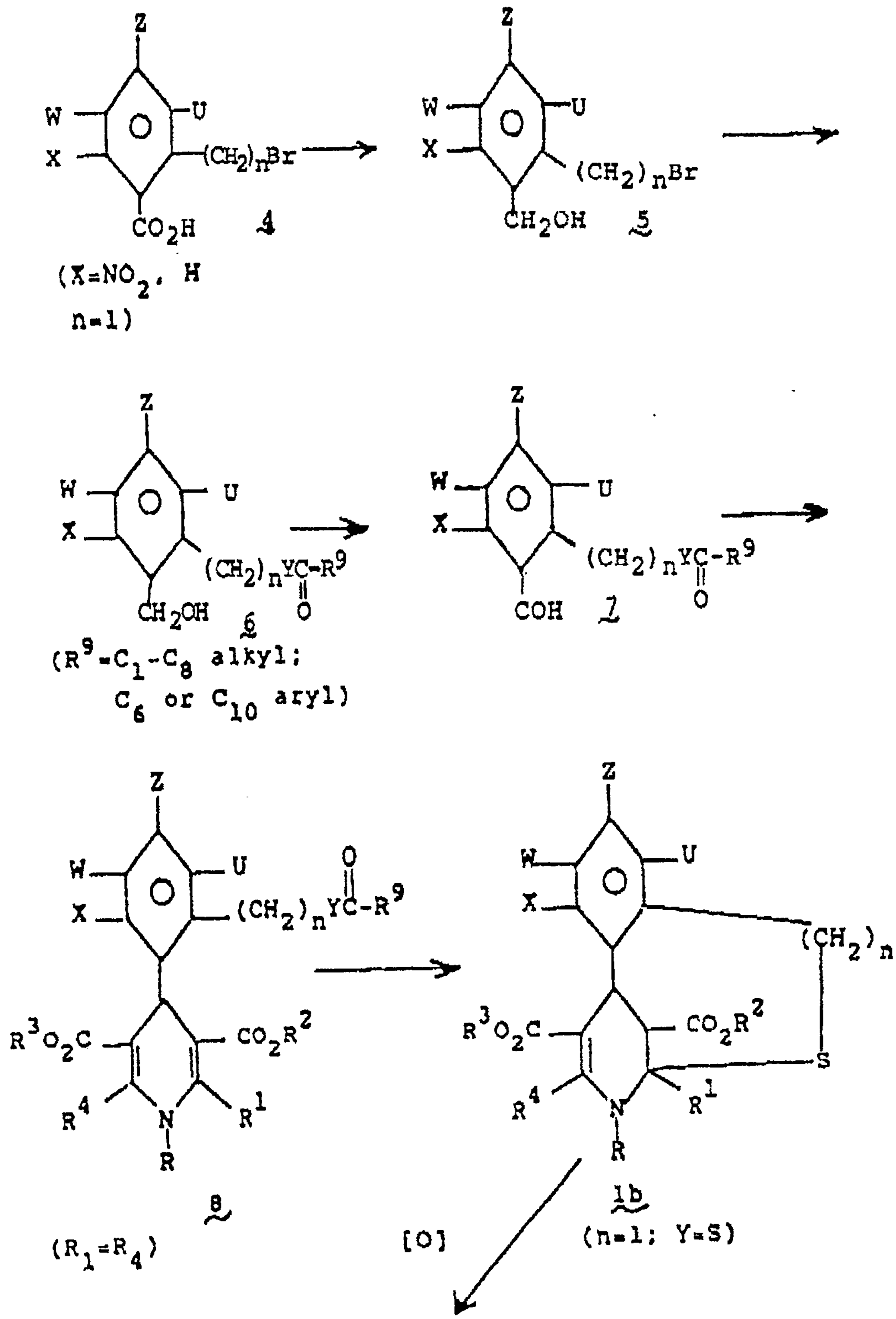

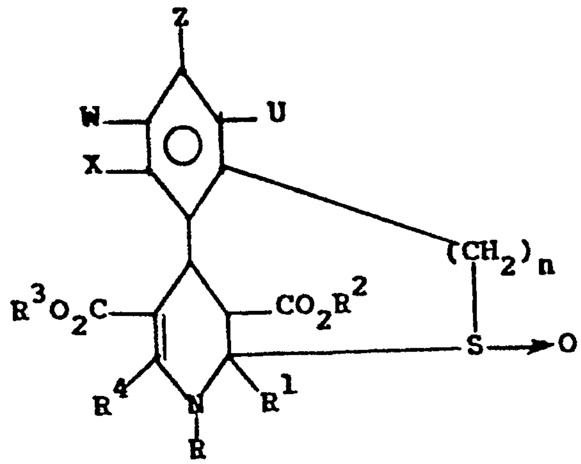

1c

(n=O; Y=S)

Method E

Step 1: As shown in Reaction Scheme III, substituted benzoic acid

4

is treated under an inert atmosphere (Ar, $N_2$) with about 1 meq of a borane complex such as borane/THF, borane/DMS, borane/piperidine (preferably borane/THF) in a polar aprotic solvent (THF, $CH_2Cl_2$, toluene; preferably THF) at about 0° -85° (preferably 25° C) for 2 hours to obtain the alcohol intermediate

5.

Step 2: Intermediate

5

is then treated with about 1.2 meq of a thioalkanoate compound, preferably potassium thioacetat

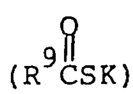

e or sodium thioacetate

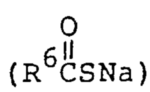

($R^6 = C_1$-$C_8$ alkyl; $C_6$ or $C_{10}$ aryl) using DMF as the solvent under the same conditions as in Step 1 to yield the methylthioacetate intermediate

6.

Step 3: Thioacetate intermediate

6

is then reacted with about 2 meq (preferably 1.5 meq) $MnO_2$, pyridinium chlorochromate (PCC), Collins reagent, dimethylsulfoxide (DMSO), oxalylchloride/triethylamine (TEA) (preferably $MnO_2$) in an organic solvent ($CH_2Cl_2$, dichloroethane, benzene, hexane; preferably $CH_2Cl_2$) at about -75° to 110° C (preferably 25° C) for about 48 hours to obtain aldehyde intermediate

7.

Step 4: Aldehyde intermediate

7

is reacted under an inert atmosphere (Ar, $N_2$) with about 1 meq each of acetoacetate

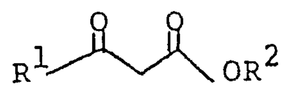

and aminocrotonate

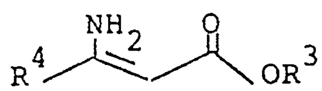

in a polar solvent (isopropanol, EtOH, $CH_3OH$, toluene, DME, THF; preferably isopropanol) at 60°-130° C, preferably the reflux temperature of the solvent to afford dihydropyridine intermediate

8,

Step 5: Upon treatment with a base ($K_2CO_3$, KOH, $Na_2CO_3$, NaOH; preferably $K_2CO_3$) in a lower alkanol solvent (methanol, ethanol; preferably methanol) under an inert atmosphere (Ar, $N_2$) at a temperature of about 25°-110° C (preferably 25° C) for 2 hours, intermediate

8

is converted to the methyl-sulfur bridged compound

1b

of the invention.

Further treatment of compound

1b

with a suitable oxidizing agent such as metachloroperoxybenzoic acid, osmium tetroxide, sodium periodate, hydrogen peroxide, or t-butyl hydroperoxide affords the sulfoxide compound

1c

of the invention.

REACTION SCHEME IV, METHOD F

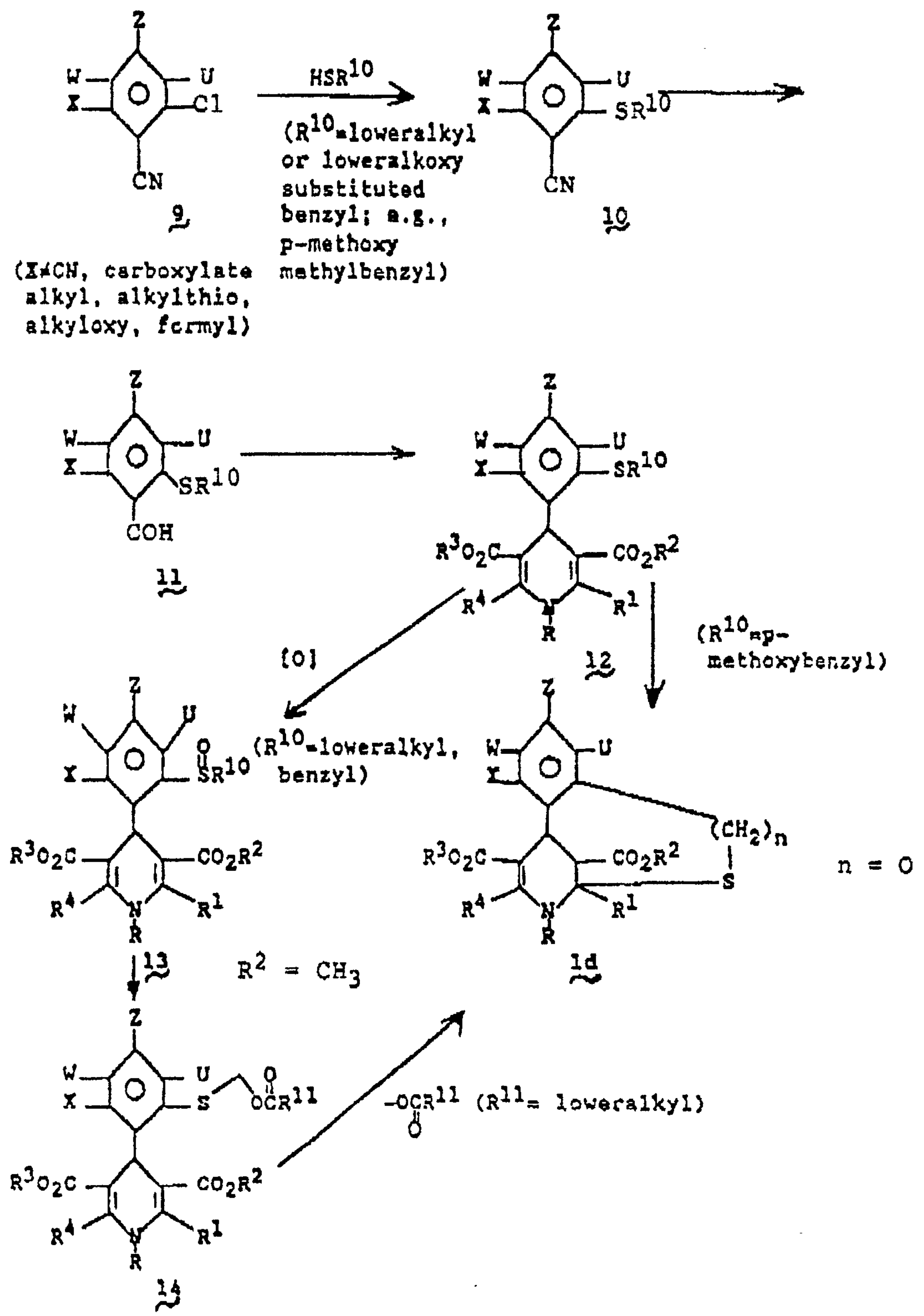


Method F

As shown in Reaction Scheme IV, substituted halobenzonitrile

9

is treated with the sulfhydryl compound, $HSR^{10}$ (1-5 meq) in a solvent (dimethylformamide (DMF), $CH_3OH$, EtOH, isopropanol or N-methyl-2-pyrrolidinone) in the presence of an alkali (lithium hydride, sodium hydride, potassium hydride, potassium carbonate, sodium methoxide, sodium ethoxide, potassium hydroxide) at about 0°-100°C (preferably 0°-25°C) for about 5 hours to obtain substituted thiobenzonitrile intermediate

10,

Treatment of intermediate

10

with diisobutylaluminum hydride (DIBAL) (1-2 meq) under an inert atmosphere (Ar, $N_2$) in an organic solvent ($CH_2Cl_2$, THF, hexane, toluene, dichloroethane) at about -78°C to the reflux temperature of the solvent (preferably -78° to 25°C) for about 3 hours followed by treatment with an aqueous acid (HCl, $H_2SO_4$, acetic acid) affords aldehyde intermediate

11.

Intermediate

11

is then reacted with 1 meq each of an acetoacetate and an aminocrotonate in the same solvent at a temperature of about 25°-130°C for about 12 hours to obtain dihydropyridine intermediate

12

which can then be reacted with trifluoroacetic acid and anisole (each at 5-50 meq; preferably 5 meq) either neat or in an organic solvent ($CH_2Cl_2$, dichloroethane, or $CHCl_3$), under an inert atmosphere (Ar, $N_2$) at about 0°-100°C (preferably 0°-25°C) for about 15 minutes to directly yield the sulfur bridged compound

1d,

of the invention.

Alternatively, dihydropyridine intermediate

12

is oxidized as described in Method E, Step 5, to obtain sulfonyl dihydropyridine intermediate

13

which is then treated with an alkanoic acid anhydride such as acetic anhydride

$$(-O\overset{\overset{O}{\|}}{C}R^{11})_2$$

(10-100 meq, preferably 5-50 meq) under an inert atmosphere (Ar, $N_2$) at about 80°-130°C (preferably 90°C) for about 1 hour to obtain dihydropyridine intermediate

**14**.

Upon treatment with an alkali (potassium carbonate, sodium hydride, lithium hydride, potassium hydride, potassium hydroxide, sodium hydroxide) under an inert atmosphere (Ar, $N_2$) in a lower alkanol organic solvent (ethanol, methanol) at about 25°-100°C (preferably 25°C) for about 5 hours, intermediate

**14**

affords compound

**1d**.

Compounds of Formula 1 can exist in different isomeric forms such as, for example, threo, erythro, diastereomeric, enantiomeric, and all such forms are included within the scope of this invention.

This invention relates further to pharmaceutical compositions consisting essentially of a pharmaceutically acceptable carrier and a pharmaceutically effective amount of a compound of the invention.

As indicated above, the compounds of this invention are useful as calcium entry blockers, and thus have broad pharmacological utility in that they exhibit (i) pronounced and long-lasting vasodilating effect accompanied by an energy-sparing effect on cardiac metabolism; (ii) antiarrythmic and antianginal action on cardiac muscle; (iii) vascular spasmolytic action; (iv) antihypertensive action; (v) spasmolytic action on the smooth muscle of the gastrointestinal and urogenital tracts and the cerebrovascular and respiratory system; (vi) are useful cholesterolemic and lipademic agents and for treating cerebral and peripheral circulatory disorders; (vii) protect ischemic myocardium; (viii) inhibit irritable bowel syndrome and esophagus spasm; and, (ix) inhibit migraine. Some of these compounds may also be useful cardiotonic agents.

The compounds of the present invention were found to inhibit vascular calcium contraction, reduce cardiac contractile force, inhibit tracheal calcium contraction, inhibit calcium uptake in pituitary cells, and displace tritiated nitrendepine from membranes.

The compounds of the present invention can be administered in any suitable form; e.g. orally, sublingually, transdermally, or parenterally; i.e. intravenously, or interperitoneally. Thus, the compounds can be offered in a form (a) for oral administration e.q. as tablets in combination with other compounding ingredients customarily used such as talc, vegetable oils, polyols, benzyl alcohols, gums, gelatin, starches and other carriers; dissolved or dispersed or emulsified in a suitable liquid carrier; in capsules or encapsulated in a suitable encapsulating material; or (b) for sublingual administration; e.g., nitroglycerine tablets, or lactose tablets for rapid dissolution or high molecular weight methylcellulose tablets, or carboxymethylcellulose tablets for slower, time-releasing delivery; or, (c) for parenteral administration e.g. dissolved or dispersed in a suitable liquid carrier or emulsified. The ratio of active compound to compounding ingredients i.e. carrier, or diluent will vary as the dosage form requires. Whatever form is used, the amount of compound of the present invention administered should be sufficient to achieve the pharmaceutical and/or therapeutic effect desired or required in the patient. Generally, doses of the compounds of the invention of from about 30 to about 3000 mg per day may be used, preferably about 100 to about 1000 mg per day. Dosages may be single or multiple depending on the daily total required and the unit dosage administered. Of course, the dose will vary depending upon the nature and severity of disease, weight of the patient, and other factors which a person skilled in the art will recognize.

It is often advantageous to administer compounds of this invention in combination with angiotensin converting enzyme inhibitors and/or antihypertensives and/or diuretics and/or β-blocking agents, and/or cardiotonic agents. For example, the compounds of this invention can be given in combination with such

compounds as enalapril, hydralazine hydrochloride, hydrochlorothiazide, methyldopa, timolol, or digitalis, as well as admixtures and combinations thereof.

Typically, the individual daily dosages for these combinations can range from about one-fifth of the minimally recommended clinical dosages to the maximum recommended levels for the entities when they are given singly. Naturally, these dose ranges can be adjusted on a unit basis as necessary to permit divided daily dosages and, as noted above, can be varied depending on the nature and severity of the disease, weight of patient, special diets and other factors.

## DETAILED DESCRIPTION OF THE INVENTION

The following Examples are provided to further illustrate the best mode currently known for obtaining the compounds of the invention, but are not to be construed as being limitative of the invention. Unless otherwise indicated, all temperatures are in ° C.

### EXAMPLE 1

Diethyl 3,6-dihydro-2,4-dimethyl-8-nitro-2,6-methano-2H-1,3-benzoxazocine-5,11-dicarboxylate (18&19)

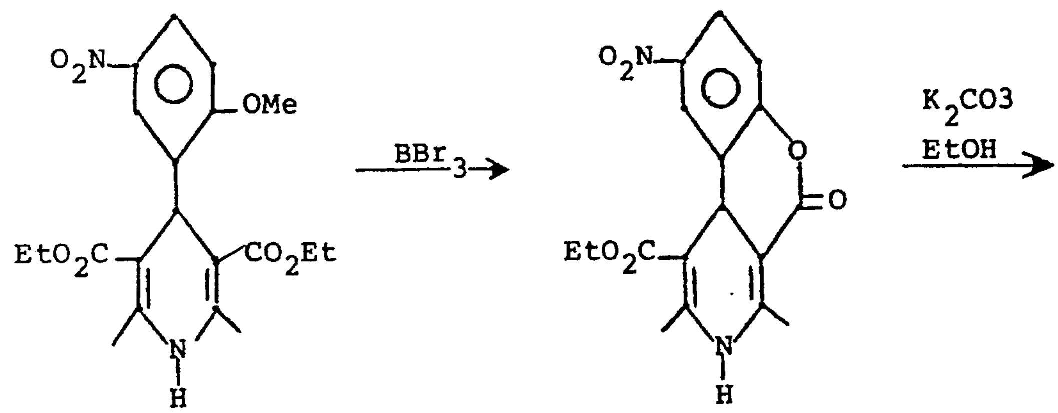


15 16

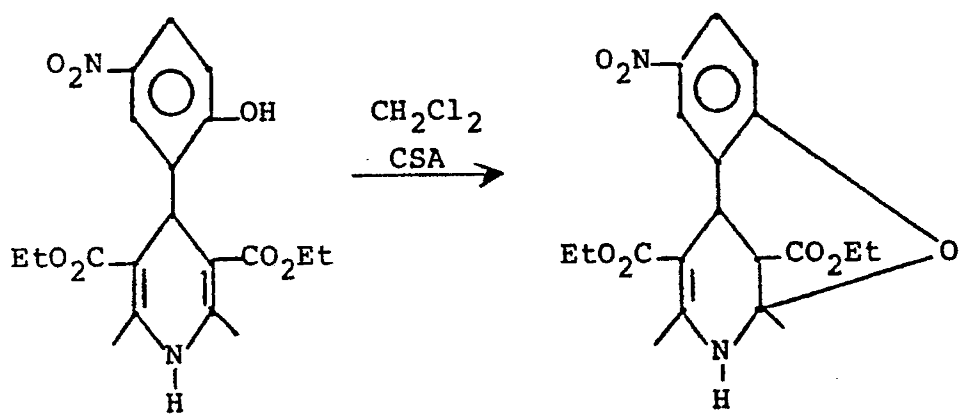


17 18&19

Step 1: Ethyl 3,10-B-dihydro-2,4-dimethyl-9-nitro-5-oxo-5H-(1)-benzopyrano-(3,4-c)pyridine-1-carboxylate

A stirred solution of 404 mg (1 mm) of diethyl 2,6-dimethyl-4-(2-methoxy-5-nitrophenyl)-1,4-dihydropyridine-3,5-dicarboxylate

15

in 20 mL of dry $CH_2Cl_2$ cooled to -78°C under an Ar atmosphere was treated dropwise with 1.25 mL of 1.0 M $BBr_3$ in $CH_2Cl_2$. The cooling bath was removed and the mixture was allowed to reach 25°C. After 20 minutes at 25°C, the reaction was quenched with saturated aqueous $NaHCO_3$ and extracted with $CH_2Cl_2$. The combined organic phases were washed with 5% aqueous $NaHCO_3$, and brine, dried (anhydrous $MgSO_4$), filtered, concentrated and recrystallized (5% EtOH in $CH_2Cl_2$) to afford 320 mg (93%) of ethyl 3,10B-dihydro-2,4-dimethyl-9-nitro-5-oxo-5H-(1)-benzopyrano(3,4-c)pyridine-1-carboxylate

16.

'H NMR (360 MHz, DMSO), δ=1.11 (t, J-7.5 Hz, 3H, $CH_2CH_3$); =2.05, 2.31 (s, 3H each, $CH_3$); 4.04, 4.21 (dq, J=10.5 and 7.5Hz, 1H each, $OCH_2$); 4.88 (s, 1H, ArCH); 7.34 (d, J=9.0Hz, 1H, ArH); 7.51 (dd, J-3,10 Hz, 1H, ArH); 8.15 (dd, J=3.0, 9.0Hz, 1H, ArH); 9.19 (bs, 1H, N-H).

Step 2: A solution of 360 mg (1.05 mm) of pyridine

16

in 30 ml of absolute ethanol under Ar atmosphere was stirred with 1.20 g of powdered anhydrous $K_2CO_3$ at 25°C for 12 hr. This was neutralized with 30 ml of saturated aqueous $NH_4Cl$ and extracted with $CH_2Cl_2$ -(200 ml). The organic portion was washed with pH 7 phosphate buffer (20 mL) and brine, dried (anhydrous $Na_2SO_4$), filtered and concentrated to give 420 mg of crude phenol

17.

Step 3: The crude phenol

17

was dissolved in anhydrous $CH_2Cl_2$ and stirred under Ar with 15 mg of dl-camphorsulfonic acid for 3 hours at 25°C. The mixture was then neutralized by washing with pH 7 phosphate buffer and brine, dried (anhydrous $MgSO_4$), filtered, concentrated to give 400 mg of crude mixture of 2 cyclized products. This was then flash chromatographed (silica gel, 75% ether in hexane) to separate the fast diastereomer

18

(100 mg) and the slower diastereomer. This slower product was further purified by preparative thick layer chromatography (PTLC) (silica gel, 2.5% acetone in $CH_2Cl_2$) and the product crystallized from ether/hexane; yield, 100 mg of

19.

18

'H NMR (360 MHz, $CDCL_3$) δ=1.25, 1.42 (t, J=7.5 Hz, 3H each, $CH_2CH_3$); δ=1.94 (s, 3H, $OCCH_3$); δ=2.24 (s, 3H, $CH_3$); 2.94 (dd, J=2.8, 0.8Hz, 1H, $CHCO_2$); 4.08-4.36 (bm, 4H, $OCH_2$); 4.67 (d, J=2.8Hz, 1H, Ar-CH); 5.05 (1H, N-H)); 6.86 (d, J=9.0 Hz, 1H, ArH); 7.98 (dd, J-3.0, 9.0 Hz, 1H, Ar-H); 8.32 (d, J=3.0Hz, 1H, ArH)

m.p. 159-160°C.

19

'H NMR (360 MHz, $CDCl_3$) δ = 1.15, 1.43 (t, J = 7.5Hz, 3H each, $CH_2CH_3$), 1.89 (s, 3H, $CH_3C$-O), 2.25 (s, 3H, $CH_3$); 2.91 (d, J = 2.4 Hz, 1H, $CHCO_2$), 4.09 (dq, J = 1.0, 7.5 Hz, 2H, $OCH_2CH_3$); 4.18, 4.34 (dq, J = 10.5, 7.5 Hz, 1H each, $OCH_2CH_3$); 4.53 (d, J = 2.4Hz, 1H, ArCH); 4.95 (bs, 1H, NH); 6.87 (d, J = 9.0Hz, 1H, ArH); 7.98 (dd, J = 3.0, 9.0Hz, 1H, ArH); 8.31 (d, J-3.0Hz, 1H, ArH).
m.p. 157-8° C.

17

'H NMR (360 MHz, $CDCl_3$) δ = 1.21 (t, J = 7.5 Hz, 6H, $CH_2CH_3$); 2.42 (s, 6H, $CH_3$); 4.15 (m, $OCH_2$); 5.07 (s, 1H, ArCH); 5.97 (bs, 1H, NH); 6.87 (d, J = 9.0Hz, 1H, ArH); 7.89 (d, J = 3.0Hz, 1H, ArH); 7.98 (dd, J = 3.0, 9.0 Hz, 1H, ArH); 9.92 (s, 1H, OH).

EXAMPLE 2

Diethyl 4,5-dihydro-3,5-dimethyl-1,5-methano-1H-naphth(1,2-G)(1,3)oxazocine-2,13-dicarboxylate (21&22)

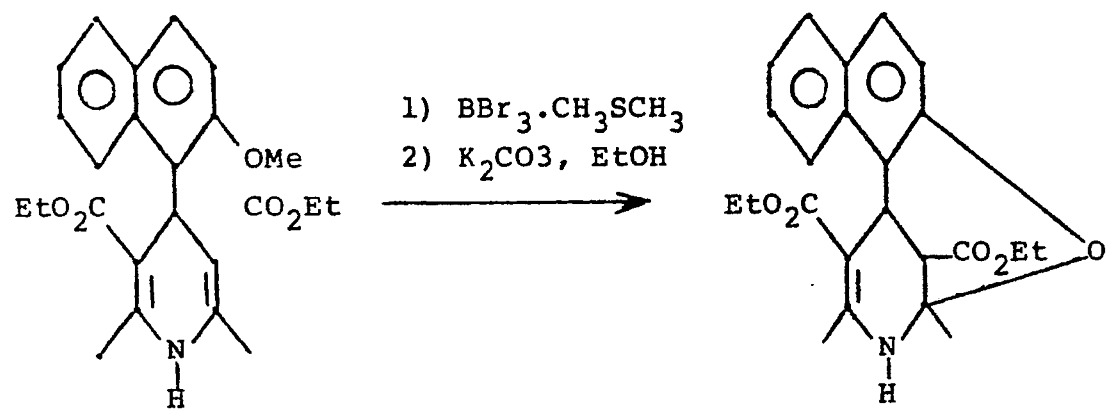


20

21 & 22

A solution of 2.25 g (5.5 mm) of diethyl 1,4-dihydro-4-(2-methoxy-1-naphthalenyl)-2,6-dimethyl-3,5-pyridinedicarboxylate

20

in 12.0 mL of dry 1,2-dichloroethane was stirred under Ar at 25° C and treated with 2.40 g (7.68 mm) of $BBr_3.CH_3SCH_3$ complex. After stirring at 25° C for 20 hours, 10.0 g of anhydrous powdered $K_2CO_3$ and 100 mL of absolute ethanol were added. Stirring was continued for 60 hours at 25°. The reaction was quenched with saturated aqueous $NH_4Cl$ and extracted with 500 ml of $CH_2Cl_2$. The organic layer was washed with pH 7 phosphate buffer and brine, dried (anhydrous $Na_2SO_4$), filtered, evaporated and flash chromatographed (silica gel, 2% acetone in $CH_2Cl_2$).

The separated diastereomers,

21

and

22,

were crystallized from ether/hexane; yield: fast

(21)

500 mg; slow

(22)

100 mg.

21

Fast: 'H NMR (360 MHz, $CDCl_3$) $\delta$ = 1.29, 1.38 (t, J = 7.0Hz, 3H each, $CH_3CH_2$); 1.96 (s, 3H, $CH_3$-C-O); 2.18 (s, 3H, $CH_3$); 3.06 (dd, J = $H_2O$, 1.0Hz, 1H, $HCCO_2$); 4.07-4.33 (m, 4H, $CCH_2CH_3$), 4.97 (bs, 1H, NH); 5.28 (d, J = 4.0Hz, 1H, ArCH); 7.02 (d, J = 9.0Hz, 1H, Ar-H); 7.33 (dt, J = 1.0, 7.5 Hz, 1H, ArH); 7.48 (dt, J = 1.0, 7.5 Hz, 1H, ArH); 7.61 (d, J = 9.0 Hz, 1H, ArH); 7.75 (bd, J = 7.5 Hz, 1H, ArH); 8.55 (d, J = 9.0 Hz, 1H, ArH).
m.p. 180-190 ° C.

22

Slow: 'H NMR (300 MHz, $CDCl_3$) $\delta$ = 1.00, 1.39 (t, J = 7.5 Hz, 3H each, $CH_2CH_3$); 1.94 (s, 3H, $OCCH_3$); 2.36 (s, 3H, $CH_3$); 2.87 (d, J = 3.0 Hz, 1H, $HCCO_2$); 4.00 (q, J = 7.5 Hz, 2H, $OCH_2CH_3$); 4.22, 4.32 (dq, J = 10.0 Hz, 7.5 Hz, 1H each, $OCH_2CH_3$); 4.91 (bs, 1H, NH); 5.23 (d, J = 3.0 Hz, 1H, ArCH); 7.02 (d, J-7.0 Hz, 1H, ArH); 7.31 (bt, J = 7.5 Hz, 1H, ArH); 7.46 (bt, J = 7.5 Hz, 1H, ArH); 7.59 (d, J = 9.0 Hz, 1H, ArH); 7.7 (bd, J = 8.0 Hz, 1H, ArH); 8.55 (d, J = 9.0 Hz, 1H, ArH);
m.p 185-92 ° C.

EXAMPLE 3

Diethyl 3,6-dihydro-2,4-dimethyl-2,6-methano-2H-1,3-benzoxazocine-5,11-dicarboxylate (26&27)

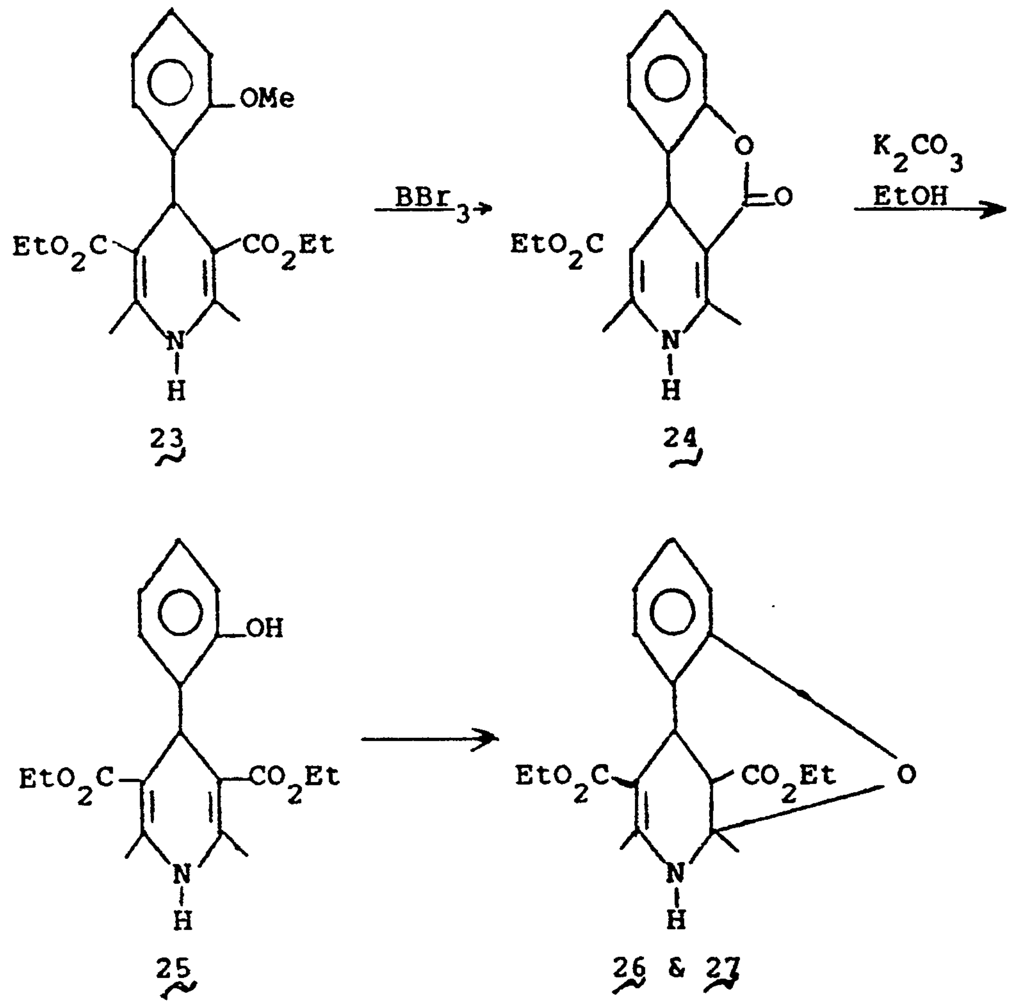


23 24

25 26 & 27

Step 1: Ethyl 3,10B-dihydro-2,4-dimethyl-5-oxo-5H-(1)benzopyrano(3,4-c)pyridine-1-carboxylate (24)

36 mL of $BBr_3$ in $CH_2Cl_2$ (1.0 M, 36 mm) was added dropwise to a cold (-78° C) stirred solution of

23,

diethyl-1,4-dihydro-4-(2-methoxyphenyl)-2,6-dimethyl-3,5-pyridinedicarboxylate, (10.77 g, 30 mm) in 60 ml of dry $CH_2Cl_2$ under an Ar atmosphere. After stirring an additional 15 minutes at -78° C, the reaction mixture was allowed to reach 25° C and stirred 1 hour. This was quenched with saturated aqueous $NH_4Cl$ and extracted with $CHCl_3$. The organic portion was washed with pH 7 phosphate buffer and brine, dried (anhydrous $MgSO_4$), filtered and evaporated to give a solid which was washed with ether and dried 12 hours/40° C/ $P_2O_5$/1 mm; yield 9.00 g of yellow solid

24

(99%). This was used without further purification in Step 2.

Analytical sample was obtained by recrystallization from $CH_2Cl_2$.

24

'H NMR (360 MHz, $CDCl_3$) $\delta$ = 1.95 (t, J = 7.5 Hz, 3H, $CH_2CH_3$); 2.12, 2.34 (s, 3H each, $CH_3$); 4.19 (m,

2H, $OCH_2$); 4.91 (s, 1H, ArCH); 5.47 (bs, 1H, N-H); 6.87 (d, J=7.5 Hz, 1H, ArH); 7.07 (t, J=7.5 Hz, 1H, ArH); 7.09 (d, J=7.5 Hz, 1H, ArH); 7.20 (t, J=7.5 Hz, 1H, ArH).

Ir (KBr) =1690, 1615 $cm^{-1}$.

m.p. 238-240 ° C.

Step 2: Lactone

24

(9.00 g) in 150 mL of absolute ethanol was vigorously stirred under Ar with 30 g of powdered anhydrous $K_2CO_3$ for 48 hours. This was treated with saturated aqueous $NH_4Cl$ and extracted with $CH_2Cl_2$. The organic portion was washed with pH 7 phosphate buffer and brine, dried (anhydrous $MgSO_4$), filtered, evaporated and flash chromatographed (silica gel, 60% ether in hexane) to afford

26

and

27.

26

Fast: 'H NMR (360 MHz, $CDCl_3$) $\delta$=1.23, 1.34 (t, J=7.5Hz, 3H each, $CH_2CH_3$); 1.89 (s, 3H, $OCCH_3$); 2.20 (s, 3H, $CH_3$); 2.95 (dd, J=3.0, 1.0 Hz, 1H, $CHCO_2$); 4.04-4.26 (m, 4H, $CH_2CH_3$); 4.61 (d, J=3.0Hz, 1H, ArCH); 4.97 (bs, 1H, N-H); 6.79 (d, J=7.5Hz, 1H, Ar);; 6.84 (dt, J=1.0, 7.5Hz, 1H, ArH); 7.07 (dt, J=1.0, 7.5 Hz, 1H, ArH); 7.36 (dd, J=1.6, 7.5 Hz, 1H, ArH).

m.p. 159-60 ° C.

27

Slow: 'H NMR (360 MHz, $CDCl_3$) $\delta$=1.11, 1.37 (t, J=7.5 Hz, 3H each, $CH_2CH_3$); 1.87 (s, 3H, $OCCH_3$); 2.18 (s, 3H, $CH_3$); 2.79 (d, J=3.0 Hz, 1H, $HCCO_2$); 4.06 (q, J=7.5 Hz, 2H, $OCH_2$); 4.18, 4.25 (dq, J=10.5, 7.5 Hz, 1H each, $OCH_3$), 4.50 (d, J=3.0Hz, 1H, ArCH); 4.84 (bs, 1H, NH); 6.79 (d, J=8.0Hz, 1H, ArH); 6.84 (dt, J=1.0, 8.0 Hz, 1H, ArH); 7.06 (dt, J-10, 8.0 Hz, H, ArH); 7.34 (dd, J=1.0, 8.0 Hz, 1H, ArH).

m.p. 100-102 ° C.

25

'H NMR (360 MHz, $CDCl_3$) $\delta$=1.19 (t, J=7.5 Hz, 6H, $CH_2CH_3$); 2.36 (s, 6H, $CH_3$); 4.12 (bm, 4H, $OCH_2$); 5.09 (s, 1H, ArCH); 5.78 (bs, 1H, NH); 6.79 (t, J=7.5 Hz, 1H, ArH); 6.88 (d, J=7.5 Hz, 1H, ArH); 7.04 (d, J=7.5 Hz, 1H, ArH); 7.06 (t, J=8.0 Hz, 1H, ArH); 8.72 (s, 1H, O-H).

m.p. 165-9 ° C.

EXAMPLE 4

Diethyl 8,10-dichloro-3,6-dihydro-2,4-dimethyl-2,6-methano-2H-1,3-benzoxazocine-5,11-dicarboxylate (31&32)

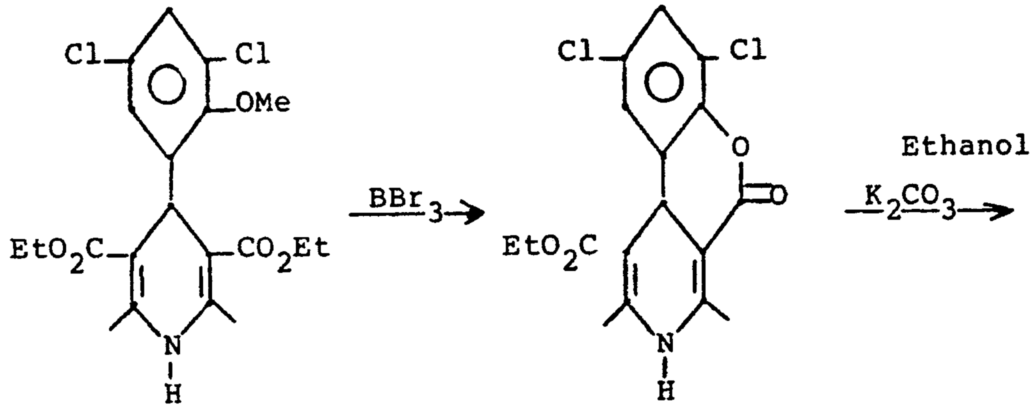


28 29

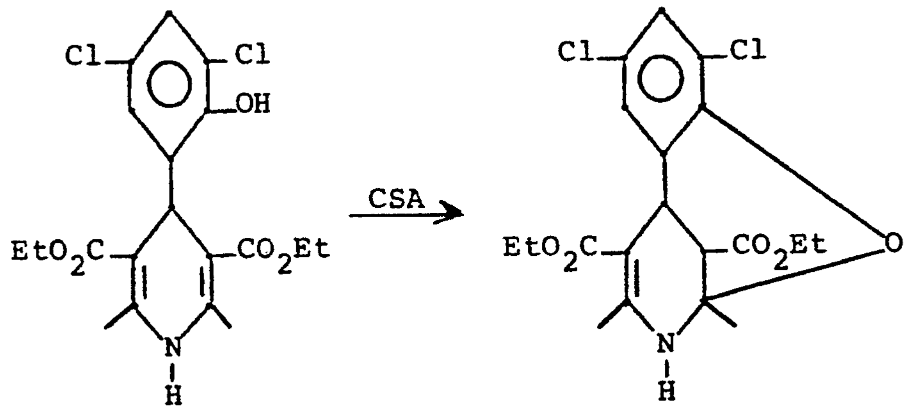


30 31 & 32

The same procedure was used as in Example 1 to obtain compounds 31 and 32.

Lactone:

29

'H NMR (360 MHz, $CDCl_3$) $\delta = 1.20$ (t, J = 7.5 Hz, 3H, $CH_2CH_3$); 2.14, 2.37 (s, 3H each, $CH_3$); 4.13, 4.27 (dq, J = 7.5, 10.0 Hz, 1H each, $OCH_2$); 4.88 (s, 1H, ArCH); 5.83 (bs, 1H, N-N); 6.72, 7.27 (m, 1H each, ArH).
IR (KBr) = 1680 $cm^{-1}$

Phenol:

30

'H NMR (360 MHz, $CDCl_3$) $\delta = 1.21$ (t, J = 7.5 Hz, 6H, $CH_2CH_3$); 2.38 (s, 6H, $CH_3$); 4.14 (m, 4H, $OCH_2$); 5.08 (s, 1H, ArCH); 5.81 (bs, 1H, N-H); 6.85 (d, J = 3.0 Hz, 1H, Ar-H); 7.16 (d, J = 3.0 Hz, 1H, ArH); 9.31 (s, 1H, OH).
m.p. 178-9 °C.

31

Fast:'H NMR (360 MHz, $CDCl_3$/DMSO (3/1)) $\delta$ = 1.23, 1.34 (t, J = 7.5 Hz, 3H each, $CH=CH_3$); 1.94 (s, 3H, $OCCH_3$); 2.21 (s, 3H, $CH_3$); 2.91 (dd, J = 3.0, 0.8 Hz, 1H, $HCCO_2$); 4.04-4.28 (m, 4H, $OCH_2$); 4.55 (d, J = 3.0 Hz, 1H, ArCH); 7.03 (bs, 1H, N-H); 7.09 (d, J = 3.0 Hz, 1H, ArH); 7.25 (d, J = 3.0 Hz, 1H, ArH).
m.p. 190-2° C.

32

Slow: 'H NMR (360 MHz, $CDCl_3$ $\delta$ = 1.15, 1.37 (t, J = 7.5 Hz, 3H each, $CH_2CH_3$); 1.91 (s, 3H, $OCCH_3$); 2.21 (s, 3H, $CH_3$); 2.83 (d, J = 3.0, 1H, $HCCO_2$); 4.09 (q, J = 7.5 Hz, 2H, $OCH_2$); 4.17, 4.28 (dq, J-7.5, 11.0 Hz, 1H each, $OCH_2$); 4.46 (d, J = 3.0 Hz, 1H, ArCH); 4.96 (bs, 1H, NH); 7.13 (d, J = 3.0 Hz, 1H, ArH); 7.26 (d, J = 3.0 Hz, 1H, ArH).
m.p. 199-203° C.

EXAMPLE 5

Diethyl 3,6-dihydro-2,4-dimethyl-2,6-methano-2H-1,3-benzothiazocine-5,11-dicarboxylate (36&37)

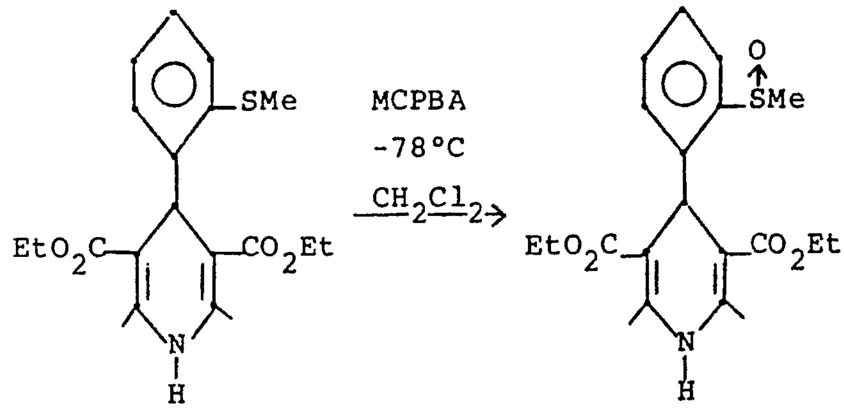


33 34

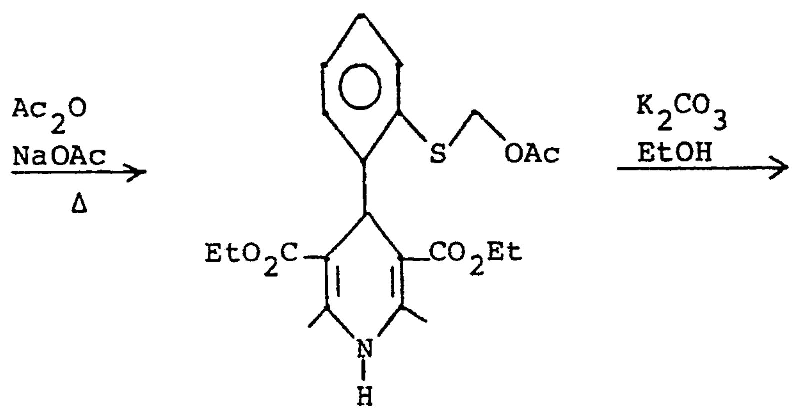


35

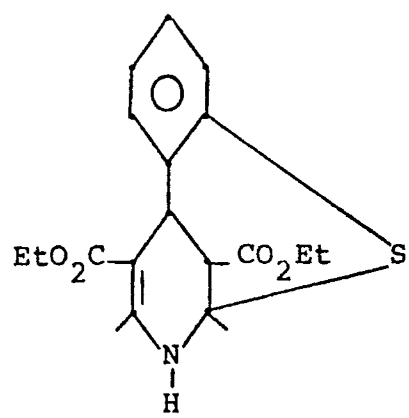

36 & 37

Step 1: Diethyl 1,4-dihydro-2,6-dimethyl-4-[2-(methylsulfinyl)-phenyl]-3,5-pyridinedicarboxylate (34)

A solution of 1.092 g (2.91 mm) of diethyl-2,6-dimethyl-4-(2-methylthiophenyl)-1,4-dihydropyridine-3,5-dicarboxylate

33

in 35 mL of dry $CH_2Cl_2$ was stirred at -78°C under Ar and treated with 650 mg (3.20 mm) of m-chloroperbenzoic acid. After 1 hour the reaction was diluted with $CH_2Cl_2$ (150 mL) and washed with 10% aqueous sodium thiosulfate, $H_2O$, and brine, dried (anhydrous $MgSO_4$), filtered and concentrated to give 1.10 g of crude sulfoxide

34

(100%) used without purification for the following step.

Analytical sample was obtained by crystallization from MeOH, m.p. 232-3° C. Diethyl-1,4-dihydro-2,6-dimethyl-4-(2-(methylsulfinyl)phenyl)-3,5-pyridinedicarboxylate

(34).

'H NMR (360 MHz, $CDCl_3$) $\delta$ = 1.20, 1.22 (t, J = 7.5 Hz, 3H, $CH_2CH_3$); 2.30, 2.32 (s, 3H each, $CH_3$); 2.82 (s, 3H, $S(O)CH_3$); 4.00-4.24 (m, 4H, $CH_2CH_3$); 5.45 (s, 1H, ArCH); 5.91 (bs, 1Hz, NH); 7.38 (m, 3H, ArH); 8.00 (m, 1H, ArH).
m.p. 232-3° C.

Step 2: Diethyl-4-[2-[[(acetyloxy)methyl]thio]phenyl]-1,4-dihydro-2,6-dimethyl-3,5-pyridinedicarboxylate (35)

A suspension of 3.31 g (8.46 mm) of sulfoxide

34

and NaOAc (anhydrous, 1.10 g) in 9.1 mL of acetic-anhydride was stirred at reflux for 40 minutes. The acetic anhydride was removed at reduced pressure, and the residue diluted with 250 mL of $CH_2Cl_2$ and 50 mL of $H_2O$. The organic portion was washed with 10% aqueous HCl, 5% aqueous $NaHCO_3$, and brine, dried (anhydrous $MgSO_4$), filtered, evaporated, and flash chromatographed (silica gel, 30% EtOAc in hexane) to give acetate

35

(1.60 g, 44%).

An analytical sample was obtained by recrystallization from ether/hexane.

35 'H NMR (360 MHz, $CDCl_3$) $\delta$ = 1.19 (t, J = 7.0 Hz, 6H, $CH_2CH_3$); 2.14 (s, 3H, $CH_3C(O)$); 2.28 (s, 6H, $CH_3$); 3.99-4.14 (m, 4H, $OCH_2$); 5.40 (s, 2H, $C(O)CH_2S$); 5.42 (s, 1H, ArCH); 5.56 (bs, 1H, NH); 7.13 (dq, J = 2.0, 7.5 Hz, 1H, ArH); 7.14 (dq, J = 2.0, 7.5 Hz, 1H, ArH); 7.37 (dd, J = 7.5, 2.0 Hz, 1H, ArH); 7.49 (dd, J = 7.5, 2.0 Hz, 1H, ArH).
m.p. 117-118° C.

Step 3: Diethyl-3,6-dihydro-2,4-dimethyl-2,6-methano-2H-1,3-benzothiazocine-5,11-dicarboxylate (36&37)

An absolute ethanol (30 mL) solution of 1.00 g (2.31 mm) of

35

was degassed with Ar and treated with anhydrous $K_2CO_3$ (2.20 g). After 3 hours of stirring under Ar, saturated aqueous $NH_4Cl$ was added and the mixture extracted with $CH_2Cl_2$. The organic portions were washed with $H_2O$ and brine, dried (anhydrous $MgSO_4$), filtered and concentrated. Flash chromatography (silica gel, 2.5% acetone in $CH_2Cl_2$) separated the two diastereomers which crystallized from ether/hexane.

36 (isomer A) Fast: 'H NMR (360 MHz, $CDCl_3$) $\delta$ = 1.03 (t, J = 7.0 Hz, 3H, $CH_2CH_3$); 1.33 (t, J = 7.0 Hz, 3H, $CH_2CH_3$); 1.96 (s, 3H, $SCCH_3$); 2.23 (s, 3H, $CH_3$); 2.96 (d, J = 4.5 Hz, 1H, $HCCO_2$); 3.90-4.25 9m, 4H, $OCH_2$); 4.64 (bs, 1H, N-H); 4.67 (d, J = 4.9 Hz, 1H, ArCH); 6.92-7.05 (m, 3H, ArH); 7.44 (dd, J = 7.5, 1.0 Hz, 1H, ArH).

37 (isomer B) Slow: 'H NMR (360 MHz, $CDCl_3$) $\delta$ = 1.26, 1.32 (t, J = 7.5 Hz, 3H each, $OCH_2CH_3$); 1.90 (s, 3H, $SCCH_3$); 2.24 (s, 3H, $CH_3$); 3.02 (dd, J = 1.5, 1.7 Hz; $HCCO_2$); 4.18-4.24 (m, 4H, $OCH_2$); 4.55 (bs, 1H, ArCH); 4.75 (bs, 1H, N-H); 7.01 (m, 3H, ArH); 7.50 (d, J = 8.0 Hz, 1H, ArH).
m.p. 147-148.5° C.

EXAMPLE 6

Diethyl 1,3,4,7-tetrahydro-3,5-dimethyl-3,7-methano-2,4-benzothiazonine-6,12-dicarboxylate (42&43)

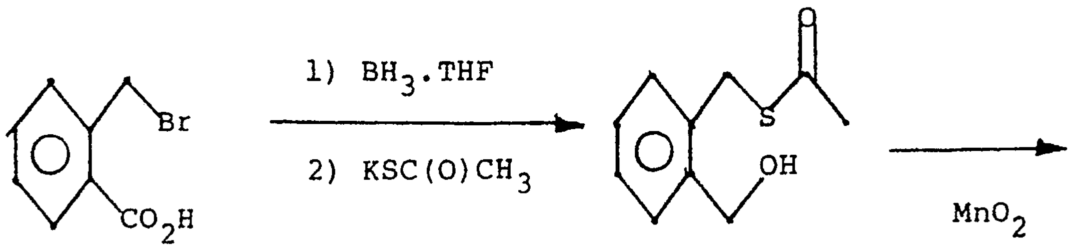


38 39

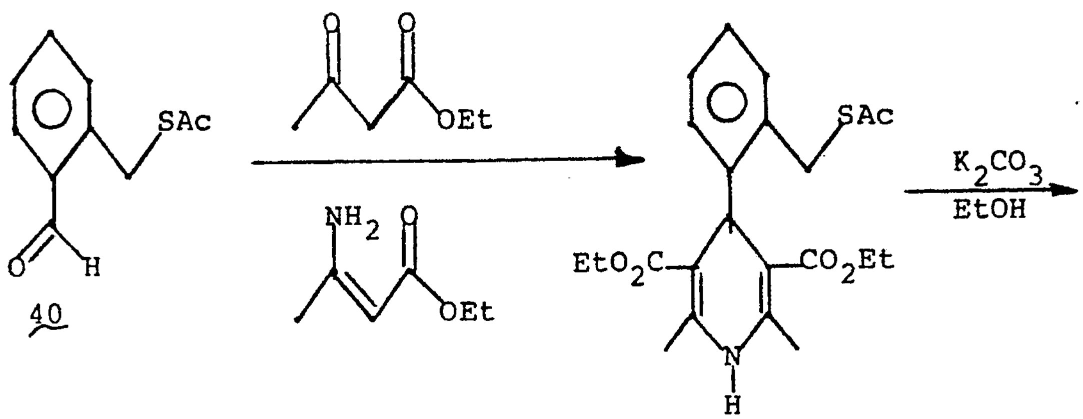


40 41

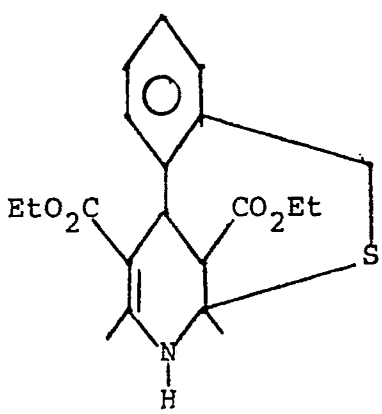

42 & 43

Step 1: Diethyl 4-[2-[(acetyl)thio]phenyl]-1,4-dihydro-2,6-dimethyl-3,5-pyridinedicarboxyate (41)

A solution of 2.00 g (9.30 mm) of o-(a-bromomethyl)benzoic acid

38

(Shawnee Chem.Co.) in 10 mL of dry THF was stirred under Ar at 0 ° C while 14.6 mL (14.6 mm) of 1.0M $BH_3$.THF was added dropwise. This mixture was stirred for 1.5 hours at 0 ° C, warmed to 25 ° C, and stirred an additional 0.5 hour. Water was added at 0 ° C to quench the reaction followed by saturated aqueous sodium potassium tartrate. After extraction with ethyl acetate, the organic portion was washed with $H_2O$ and brine, dried (anhydrous $MgSO_4$), filtered and concentrated.

The crude product was treated directly with anhydrous DMF (8 mL) and stirred while 1.42 g of potassium thioacetate was added. After 10 minutes at 25 ° C, $H_2O$ was added and the mixture was extracted with ethyl acetate. The organic portion was dried (anhydrous $MgSO_4$), filtered, and evaporated to give crude alcohol

39

(2.00 g).

The crude alcohol

39

was dissolved in 100 mL of $CH_2Cl_2$ and stirred with 6.00 g of activated $MnO_2$ for 5 days. Filtration through Celite® and evaporation afforded 1.40 g of aldehyde

40.

Aldehyde

40

'H NMR (90 MHz, $CDCl_3$) $\delta = 2.25$ (s, 3H, $CH_3$ C(O)); 4.45 (s, 2H, $CH_2S$); 7.00-7.90 (m, 4H, ArH); 10.15 (s, 1H, CHO).

Alcohol Crude:

39

'H NMR (90 MHz, $CDCl_3$) $\delta = 2.25$ (s, 3H, $CH_3$ C(O)); 4.15 (s, 2H, $SCH_2$); 4.70 (s, 2H, $CH_2O$); 5.4 (s, 1H, OH); 7.0-9.2 (m, 4H, ArH).

1.40 Gram of aldehyde

40

was refluxed in isopropyl alcohol (10 mL) with 930 mg of ethyl aminocrotonate and 930 mg of ethyl acetoacetate under Ar for 24 hours. This was concentrated and flash chromatographed (silica gel, 2.5% acetone in $CH_2Cl_2$) to give: 500 mg

41.

41

'H NMR (90 MHz, $CDCl_3$), $\delta$=1.20 (t, J=7.5 Hz, 6H, $CH_2CH_3$), 2.30 (s, 6H, $CH_3CN$); 2.39 (s, 3H, $CH_3$ C-(O)); 4.15 (q, J=7.5 Hz, 4H, OCHz); 4.55 (s, 2H, $SCH_2$); 5.30 (s, 1H, ArCH); 5.60 (bs, 1H, NH; 7.00-7.40 (m, 4H, ArH).

Step 2: Diethyl 1,3,4,7-tetrahydro-3,5-dimethyl-3,7-methano-2,4-benzothiazonine-6,12-dicarboxylate (42&43)

A mixture of 5.20 g of dihyropyridine

41,

15.0 g of anhydrous potassium carbonate, and 150 mL of degassed absolute ethanol was vigorously stirred under an Ar atmosphere for 12 hours. Saturated aqueous $NH_4Cl$ was added, and the whole was extracted with ethylacetate. The organic portion was washed with $H_2O$, and brine, dried (anhydrous $MgSO_4$), filtered, and flash chromatographed (silica gel, 2% acetone in $CH_2Cl_2$) to afford

42

and

43.

42

'H NMR (360 MHz, $CDCl_3$) $\delta$= 1.12, 1.27 (t, J=7.0 Hz, 3H each, $CH_2$-$CH_3$); 1.57 (S, 3H, S-C-CH); 2.45 (s, 3H, $CH_3$); 3.33 (d, J=15.0 Hz, 1H, S-CHaHb); 3.47 (bs, 1H, H $CO_2$); 3.94 (m, 2H, $OCH_2$); 4.17 (m, 2H, $OCH_2$); 4.24 (bs, 1H, ArCH); 4.62 (bs, 1H, NH); 4.73 (d, J=15.0 Hz, 1H, S-CHaHb); 6.95 (dd, J=1.0, 7.0 Hz, 1H, ArH); 7.08 (dt, J=1.0, 7.0 Hz, 1H, ArH); 7.16 (dt, J=1.0, 7.0 Hz, 1H, ArH); 7.33 (dd, J=1.0, 7.0 Hz, 1H, ArH).

43 'H NMR (360 MHz, $CDCl_3$) $\delta$=1.14, 1.17 (t, J=7.0 Hz, 3H each, $CH_2CH_3$); 1.69 (S, 3H, $SCCH_3$); 2.42 (S, 3H, $CH_3$); 3.18 (d, J=6.0 Hz, 1H, $HCO_2$); 3.27 (d, J=15.0 Hz, 1H, SCHaHb); 3.99 (M, 2H, $CH_2CH_3$); 4.47 (bs, 1H, N-H); 4.56 d, J=15.0 Hz, 1H, SCHaHb); 4.65 (d, 5=6.0 Hz, 1H, ArCH); 6.94 (dd, J=1.0, 7.0 Hz, 1H, ArH); 7.04 (dt, J=1,0, 7.0 Hz, 1H, ArH); 7.08 (dt, J=1,0, 7.0 Hz, 1H, ArH); 7.27 (dd, J=1.0, 7.0 Hz, 1H, ArH).

EXAMPLE 7

Diethyl 1,3,4,7-tetrahydro-3,5-dimethyl-8-(trifluoromethyl)-3,7-methano-2,4-benzothiazocine-6,12-dicarboxylate (47&48)

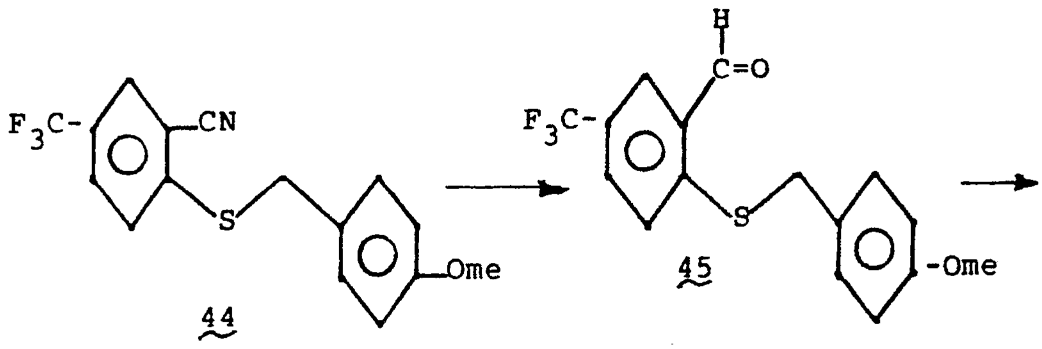

44

45

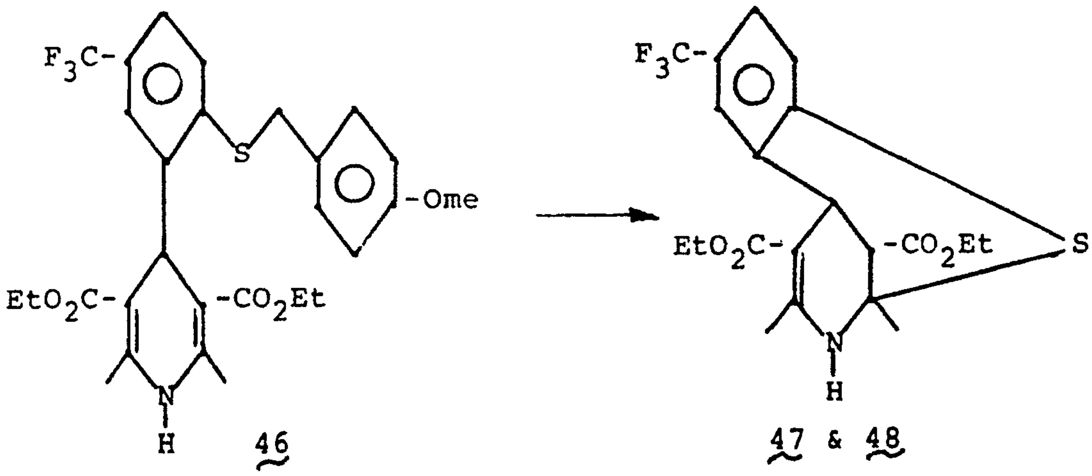

46

47 & 48

STEP 1: A cold (-78° C) anhydrous methylene chloride (90 mL) solution of nitrile

44

(28.0 g, 86.6 mm) was stirred while 87 mL of 1.0 M diisobutylaluminum hydride in Hexane (87 mm) was added dropwise over 15 minutes. This was stirred an additional 1.0 h at -78° C then allowed to reach 25° C. The mixture was recooled to -78° C and treated with 20 mL of methanol and 50 mL of saturated sodium potassium tartrate. After warming to 25° C, ethyl acetate was added and the mixture was stirred while 2.4 N HCl was added to acidify the aqueous layer. After stirring for 1 hour at room temperature, the organic portion was separated and the aqueous portion was extracted with ethyl acetate. The combined organic portions were washed with $H_2O$, and brine, dried (anhydrous $MgSO_4$), filtered and concentrated. The oil crystalized from ether/hexane (1/1) to afford 26.0 grams of a white solid

45

(92%). a-(4methoxy-a-toluenylthio)-5-(trifluoromethyl)benzaldehyde:
1H NMR (90 MHz, $CDCl_3$) $\delta = 3.75$ (S, 3H, $OCH_3$); 4.12 (S, 2H, $SCH_2$), 6.7-8.0 (multiplets, 7H, ArH); 10.15 (S, 1H, CHO).

STEP 2: 23.1 g (70.8 mm) of aldehyde

45,

9.20 g of methyl acetoacetate and 9.20 g of ethylaminocrotonate were refluxed in 30 mL of isopropylalcohol for 20 hours. After cooling to 25° C and evaporation of the solvents, a white solid precipitated from ether. This was washed with ether/hexane (1:1) to give 23.0 grams of

46.

46

diethyl-1,4-dihydro-4-(2-(4-methoxy-a-toluenylthio)5-(trifluoromethyl)phenyl)-2,6-dimethlyl-3,5-pyridine dicarboxylate

'H NMR (90 MHz, $CDCl_3$) δ 1.15 (t, J=7.5 Hz, 6H, $CH_2CH_3$); 2.18 (S, 6H, pyr.-$CH_3$); 3.70 (S, 3H, $OCH_3$); 3.95 (q, J=7.5 Hz, 4H, $OCH_2$); 4.05 (S, 2H, $SCH_2$); 5.35 (S, 1H, Aryl. CH); 5.73 (bs, 1H, N-H); 6.70-7.50 (m, 7H, Ar-H).

STEP 3: A solution of 2.20 grams of

46

was dissolved in 10 mL of anisole and degassed with Ar for 15 minutes. This was stirred while 10 mL of trifluoroacetic acid was added dropwise over 2 minutes at 25°C. After 15 minutes, the reaction was complete and the mixture diluted with $CH_2Cl_2$ and washed with $H_2O$, and saturated aqueous $NaHCO_3$, dried (anhydrous $MgSO_4$), filtered and concentrated. The two diastereomers formed were separated by flash chromatography (silica gel, $CH_2Cl_2$, then 2.5% acetone in $CH_2Cl_2$). After concentration of appropriate fractions the pure diastereomers were crystallized from ether/hexane to afford the

47

and

48

isomers.

Yield: 500 mgs of the fast diastereomer

47,

350 mgs of the slow diastereomer

48,

and 1.00 gr of a mixture of

47

and

48.

47

'H NMR ($CDCl_3$), 360 mHz) δ=1.01 and 1.35 (t, J=7.5 Hz, 3H each, $CH_2CH_3$); 1.97 (S, 3H, S-C-$CH_3$);

2.25 (S, 3H, $CCH_3$); 3.00 (d, J=4.5 Hz, 1H, HCCOz); 3.92-4.27 (m, 4H, $OCH_2$); 4.68 (d, J=4.5 Hz, 1H, ArCH); 4.72 (bs, 1H, N-H); 7.13 (d, J=7.5 Hz, 1H, ArH); 7.24 (dd, J=7.5 Hz, 1.5H, 1H, ArH); 7.69 (d, J=1.5 Hz, 1H, ArH).

48 1H NMR ($CDCl_3$, 360 MHz) $\delta$=1.27 and 1.34 (t, J=6.0 Hz, 3H each, $CH_2CH_3$); 1.93 (S, 3H, $SCCH_3$); 2.27 (S, 3H, $CH_3$); 2.97 (dd, J=3.0, 1.5 Hz, 1H, $HCCO_2$); 4.07-4.27 (m, 4H, $OCH_2$); 4.58 (bs, 1H, ArCH); 4.83 (bs, 1H, NH); 7.13 (d, J=7.5 Hz, 1H, ArH; 7.27 (dd, J=7.5, 1.5 Hz, 1H, ArH); 7.76 (d, =1.5 Hz, 1H, ArH).

EXAMPLE 8

Diethyl 1,3,4,7-tetrahydro-3,5-dimethyl-8-nitro-3,7-methano-2,4-benzothiazocine-6,12-dicarboxylate (53&54)

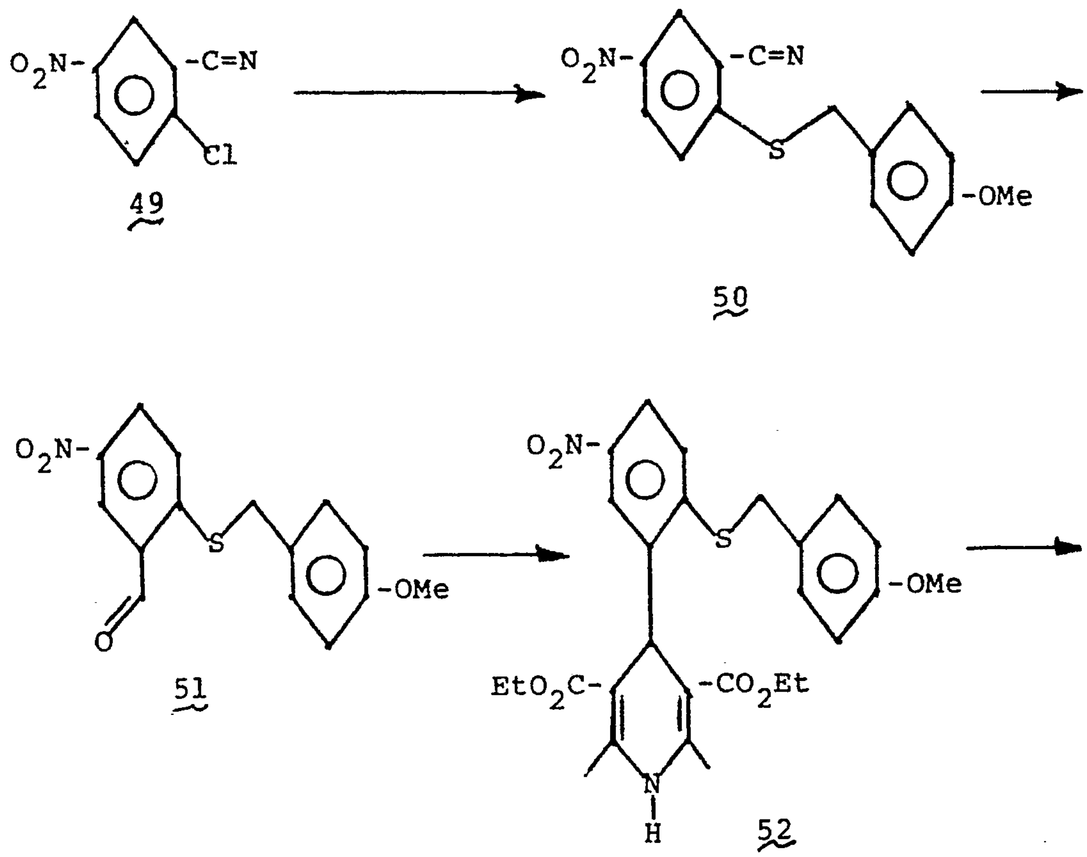

49

50

51

52

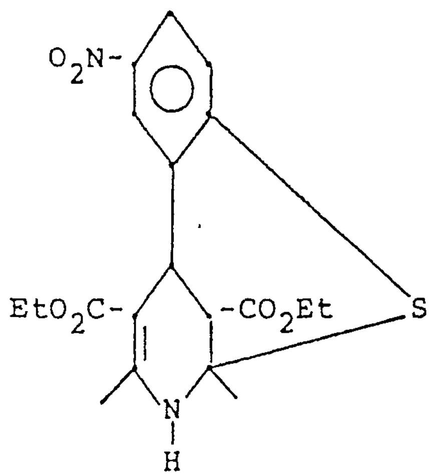

53 & 54

STEP 1: A solution of 0.84 g of p-methoxy-a-toluenethiol (5.45 mm) in 15 mL of degassed methanol cooled to 0° C was stirred while 393 mg of sodium methoxide was added. After stirring for 10 minutes, 1.03 g (5.64 mm) of 2-chloro-5-nitrobenzonitrile

49

was added in one portion and this was allowed to stir for 1 hour at 0° C, then 2 hours at 25° C. This mixture was then diluted with $H_2O$ (60 mL) and stirred 5 minutes. The solid precipitate that formed was collected by filtration and washed with $H_2O$. This was dried at 80° C (1 mm - 1.33 mbar) for 12 hours from which

49

was removed by sublimation. The remaining yellow solid, 2-(4-methoxy-a-toluenethio)-5-nitro-benzo-nitrile

50

(1.40 g, 77% yield) was used without further purification for the next step.

50 1H NMR (90 MHz, $CDCl_3$) $\delta = 3.80$ (S, 3H, $OCH_3$); 4.30 (S, 2H, $SCH_2$); 6.80-7.60 (m, 5H, ArH); 8.30 (dd, J=3.0, 9.0 Hz, 1H, ArH); 8.45 (d, J=3.0 Hz, 1H, ArH).

STEP 2: Nitrile

50

was reduced in the same manner as compound

44

(Example 7) to give aldehyde

51

as a yellow solid in 65% yield.

51

2-(4-methoxy-a-toluenethio)-5-nitrobenzaldehyde:
'H NMR (90 MHz, $CDCl_3$) $\delta = 3.80$ (S, 3H, $OCH_3$); 4.30 (S, 2H, $SCH_2$); 6.90 (d, J = 8.0 Hz, 2H, ArH); 7.39 (d, J = 8.0 Hz, 2H, ArH); 7.58 (d, J = 9.0 Hz, 1H, ArH); 8.32 (dd, J = 9.0, 3.0 Hz, 1H, ArH); 8.68 (d, J = 3.0 Hz, 1H, ArH); 10.38 (S, 1H, CHO).

STEP 3: Aldehyde

51

was condensed in a similar manner to compound

45

(Example 7) to provide dihydropyridine

52

after crystalization from ether/hexane/$CH_2Cl_2$ in 50% yield.

52

diethyl 1,4-dihydro-4-(2-(4-methoxy-a-toluenylthio)-5-nitrophenyl)-2,b-dimethyl-3,5-pyridinedicarboxylate:
'H NMR (70 MHz, $CDCl_3$): $\delta = 1.10$ (t, J = 7.5 Hz, 6H, $CH_2CH_3$); 2.22 (S, 6H, $CH_3$); 6.77 (S, 3H, $CCH_3$); 4.03 (q, J = 7.5 Hz, 4H, $OCH_2$); 4.14 (S, 2H, $SCH_2$); 5.40 (S, 1H, ArCH); 5.68 (bs, 1H, NH); 7.79 (d, J = 8.0 Hz, 2H, ArH); 7.23 (m, 3H, ArH); 7.81 (dd, J = 3.0, 9.0 Hz, 1H, ArH); 8.08 (d, J = 3.0 Hz, 1H, ArH).

STEP 4: 5.4 grams of dihydropyridine

52

was treated similarly to dihydropyridine

46

(Example 7) to provide two diastereomeric benzothiazocines

53

and

54

which were separated by flash chromatography (silica gel, 2.5% acetone in methylene chloride) to give, after crystalization, the isomers

53

and

54.

53

'H NMR (360 MHz, $CDCl_3$) $\delta = 1.07$ and 1.41 (t, J = 7.5 Hz) 3H each, $CH_2CH_3$); 1.98 (S, 3H, $SCCH_3$); 2.26 (S, 3H, $CH_3$); 3.05 (d, J = 4.5 Hz, 1H, $HCCO_2$); 3.90-4.30 (m, 4H, $OCH_2$); 4.73 (d, J = 4.5 Hz, 1H, ArCH); 4.77 (bs, 1H, NH); 7.16 (d, J = 9.0 Hz, 1H, ArH); 7.88 (dd, J = 3.0, 9.0 Hz) 1H, ArH); 8.33 (d, J = 3.0 Hz, 1H, ArH).

54

'H NMR (360 MHz, $CDCl_3$) $\delta = 1.28$, 1.39 (t, J = 7.5 Hz, 3H each, $CH_2CH_3$); 1.96 (S, 3H, $SCCH_3$); 2.28 (S, 3H, $CH_3$); 2.96 (dd, J = 2.0, 1.5 Hz, 1H, $HCCO_2$); 4.09-4.28 (m, 4H, $OCH_2$); 4.64 (bs, 1H, ArCH); 4.87 (bs, 1H, NH); 7.15 (d, J = 9.0 Hz, 1H, ArH); 7.90 (dd, J = 3.0, 9.0 Hz, 1H, ArH); 8.38 (d, J = 3.0 Hz, 1H, ArH).

EXAMPLE 9

Dimethyl 8-(2,4-difluorophenyl)-3,6-dihydro-2,4-dimethyl-2,6-methano-2H-1,3-benzoxazocine-5,11-dicarboxylate (61&62)

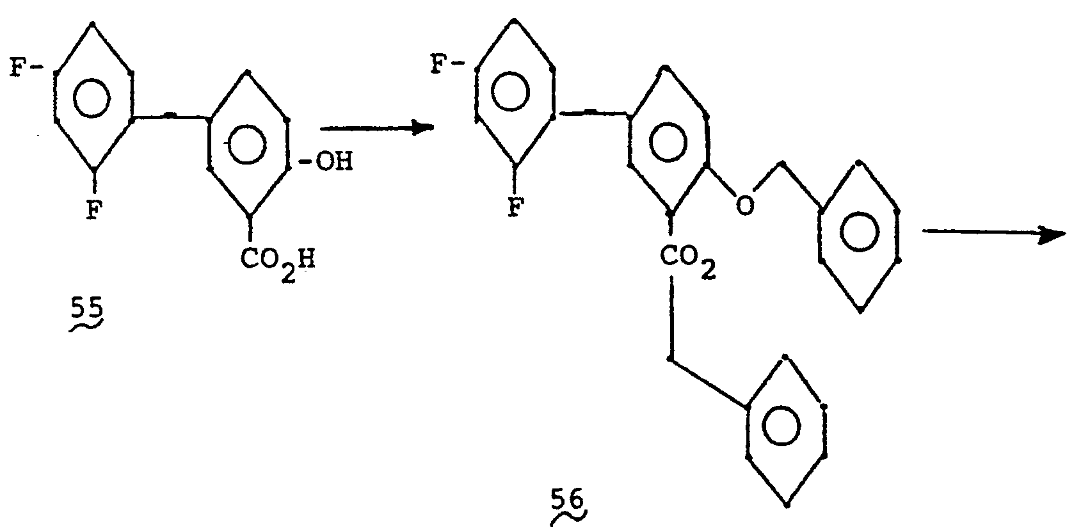

55

56

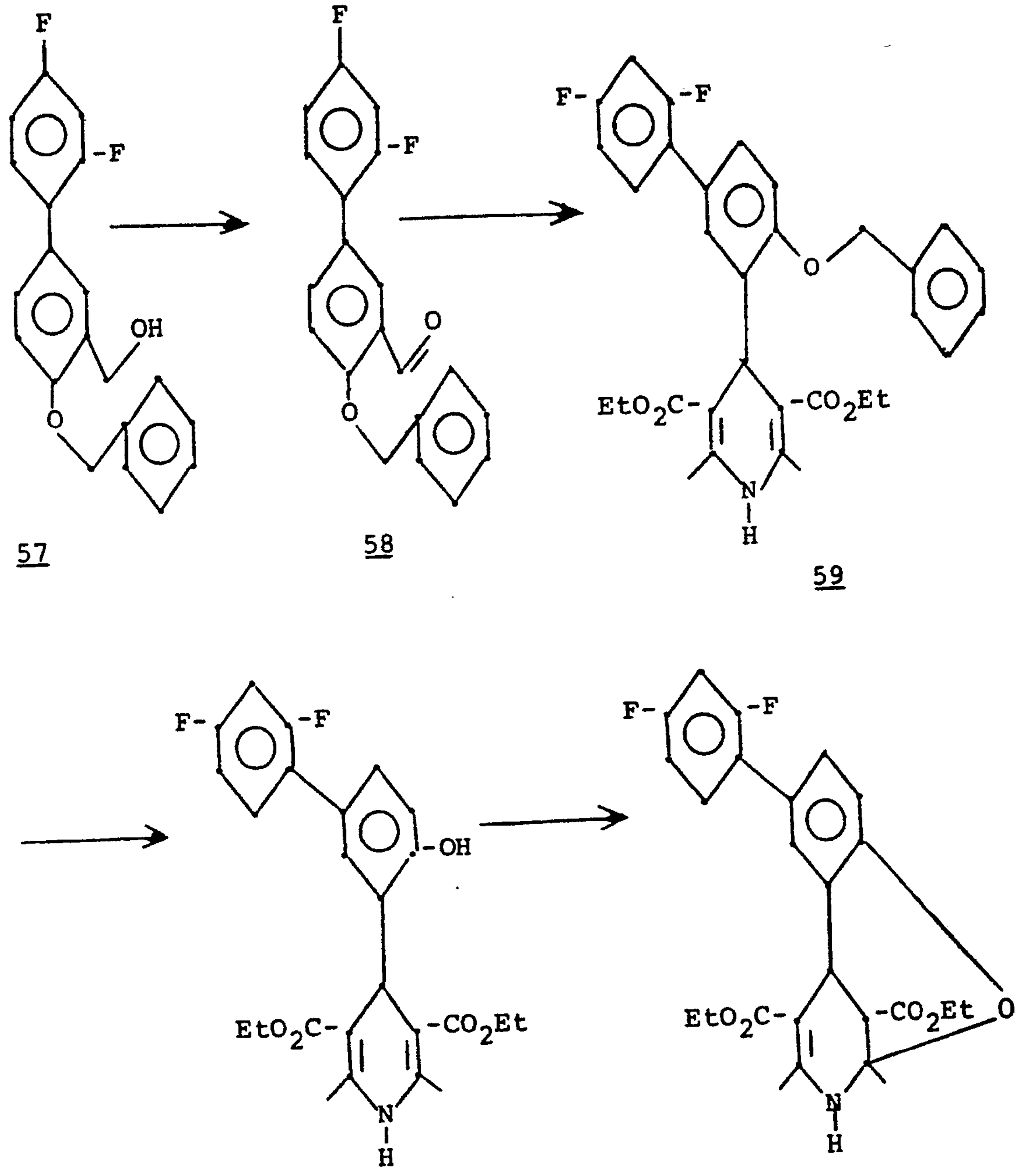

57 58 59

60

61 & 62

STEP 1: A solution of 2', 4'-difluoro-4-hydroxy (1,1'-biphenyl)-3-carboxylic acid

55

(1.00 g, 4.00 mm) in 8 mL of dimethylformamide was stirred at 25° C and treated with 5.52 g (40.00 mm) of powdered potassium carbonate and 1.2 mL of benzyl bromide (10.0 mm). After 24 hours, the mixture was diluted with $H_2O$ and extracted with ethyl acetate. The organic portion was washed with $H_2O$ and brine, dried (anhydrous $MgSO_4$), filtered and evaporated to yield, after crystallization from ether/hexane, 1.50 g of

56

as a white solid (81%).

56 benzyl 2',4'-difluoro-4-benzyloxy(1,1'-biphenyl) -3-carboxylate.

'H NMR (90 MHz, $CDCl_3$) $\delta = 5.20$ (S, 2H, $OCH_2$); 5.40 (S, 2H, O $CH_2$); 6.8-7.2 (m, 15 H, ArH); 7.97 (m, 1H, ArH).

STEP 2:

56

(7.25 g, 16.86 mm) was dissolved in 125 mL of anhydrous tetrahydrofuran and diethyl ether (1/3), stirred at 0° C under Ar, and treated with lithium aluminum hydride (641 mg, 16.86 mm). After 1 hour the reaction was quenched with 10% aqueous tetrahydrofuran and then dried (anhydrous $MgSO_4$), filtered and concentrated to give 5.25 g (92%) of a white solid

57

after recrystallization from ether/hexane.

57 2-benzyloxy-5-(2,4-difluorophenyl)benzylalcohol:

'H NMR (90 MHz, CDC ) $\delta = 2.27$ (t, J=6.0 Hz, 1H, 0H); 4.82 (d, J=6.0 Hz, 2H, $CH_2OH$); 5.18 (S, 2H, $CH_2$); 6.70-6.90 (m, 3H, ArH); 7.40 (M, 8H, ArH).

STEP 3: A methylene chloride (200 mL) solution of alcohol 57 (5.25 g) was stirred with 15.0 g of activated $MnO_2$ for 60 hours at 25° C. After filtration and concentration aldehyde

58

was obtained (3.20 g, 61%) as a white solid.

58

2',4'-difluoro-4-benzyloxy(1,1'-biphenyl)-3-carboxaldehyde.

'H NMR (90 MHz $CDCl_3$) $\delta = 5.25$ (S, 2H, $OCH_2$); 6.75-7.75 (m, 10H, ArH); 8.00 (d, J=3 Hz, 1H, ArH); 10.60 (S, 1H, CHO). m.p. 124.0-125.5° C.

STEP 4: 3.00 g of

58

was condensed under similar conditions as aldehyde

45

to give 3.00 g of dihydropyridine

59

after recrystallization from ether/hexane.

59

diethyl 1,4-dihydro-4-(2-benzyloxy-5-(2,4-difluorophenyl)phenyl)-2,6-dimethyl-3,5-pyridinedicarboxylate: 'H NMR (90 MHz, $CDCl_3$) δ = 1.10 (t, J = 7.0 Hz, 6H, $CH_2CH_3$); 2.05 (S, 6H, $CH_3$); 4.03 (q, J = 7.0 Hz, 4H, $OCH_2CH_3$); 4.80 (bs, 1H, NH); 5.00 (S, 2H, $OCH_2Ar$); 5.25 (S, 1H, ArCH); 6.90-7.50 (m, 11H, ArH).

STEP 5: An absolute ethanol solution of dihydropyridine 59 (2.66 g in 100 mL) was charged with 210 mg of 5% Pd on carbon and shaken under 1 Atm of $H_2$ for 4 days. This was filtered and concentrated to give the phenol

60

(2.40 g).

60

diethyl 1,4-dihydro-4-(5-(2,4-difluorophenyl)-2-hydroxyphenyl)-2,6-dimethyl-3,5-pyridine dicarboxylate: 1H NMR (90 MHz, $CDCl_3$) δ = 1.20 (t, J = 7.5 Hz, 6H, $CH_2CH_3$); 2.35 (S, 6H, $CH_3$); 4.10 (q, 5 = 7.5 Hz, 4H, $OCH_2$); 5.15 (S, 1H, ArCH); 6.10 (bs, 1H, NH); 6.70-7.45 (m, 6H, ArH); 9.05 (bs, 1H, OH).

STEP 6: Phenol

60

was treated in a similar manner to compound

17

(Example 1) to give, after flash chromatography (silica gel, 2.5% acetone in methylenechloride) and concentration of appropriate fractions, the two title diastereomeric benzoxazocines 61 and 62 as white solids (from ether/hexane).

61 'H NMR (360 MHz, $CDCl_2$) δ = 1.13, 1.33 (t, J = 7.5 Hz, 3H each, $CH_2CH_3$); 1.88 (S, 3H, $OCCH_3$); 2.21 (S, 3H, $CH_3$); 2.83 (d, J = 3 Hz, $HCCO_2$); 4.09 (q, J = 7.5 Hz, 2H, $OCH_2$); 4.10-4.30 (m, 2H, $OCH_2$); 4.54 (d, J = 3.0 Hz, 1H, ArCH); 4.89 (bs, 1H, NH); 6.87 (m, 3H, ArH); 7.22 (dt, J = 8.0 Hz, 2.0 Hz, 1H, ArH); 7.36 (dt, J = 7.0, 8.0 Hz, 1N, ArH); 7.53 (bs, 1H, ArH).

62 'H NMR (360 MHz, $CDCl_3$) δ = 1.25, 1.32 (t, J = 7.5 Hz, 3H each, $CH_2CH_3$); 1.93 (S, 3H, $OCCH_3$); 2.24 (S, 3H, $CH_3$); 3.00 (dd, J = 3.0, 1.5 Hz, 1H, $HCCO_2$, collapses to doublet J = 3.0 Hz upon irradiation at 4.97 ppm); 4.05-4.26 (m, 4H, $OCH_2$); 4.64 (d, J = 3.0 Hz, 1H, ArCH); 4.97 (bs, 1H, NH); 6.89 (m, 3H, ArH); 7.22 (dt, 5 = 8.0 Hz, 1.5 Hz, 1H, ArH); 7.35 (dt, J = 7.0, 9.0 Hz, 1H, ArH); 7.53 (t, J = 1.5 Hz, 1H, ArH).

EXAMPLE 10

Diethyl 3,6-dihydro-2,4-dimethyl-10-nitro-2,6-methano-2H-1,3-benzothiazocine-5,11-dicarboxylate (68&69)

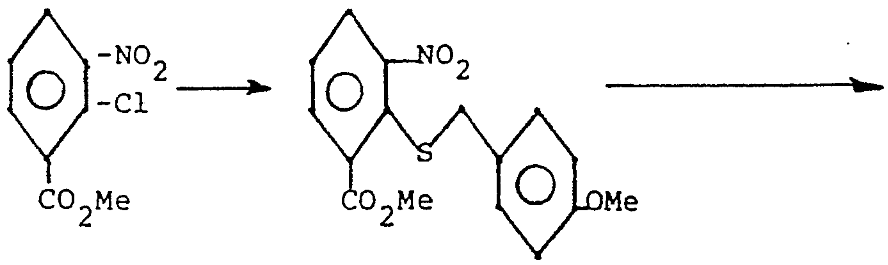

63 64

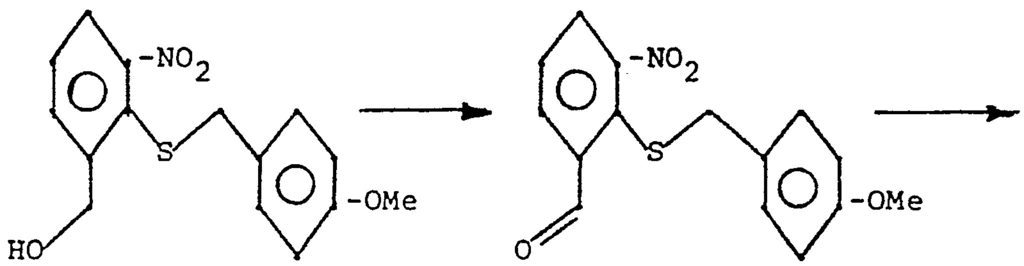

65 66

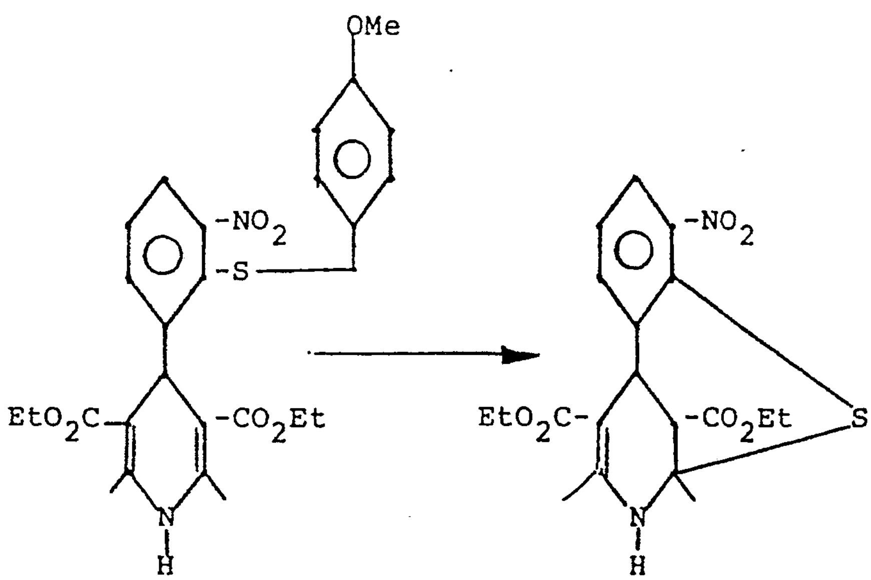

67 68 & 69

STEP 1: An anhydrous dimethylformamide (DMF) solution of p-methoxy-a-toluenethiol (1.86 mL in 15 mL of DMF) was cooled to 0°C under Ar and 106 mg (13.37 mm) of lithium hydride was added. This stirred suspension was warmed to 25°C for 20 minutes and then methyl 2-chloro-3-nitrobenzoate 63 was added in one portion. An exothermic reaction took place and was cooled to 0°C. The mixture was rewarmed to 25°C and stirred for 10 minutes, diluted with $H_2O$, and extracted with ethyl acetate. The organic portion was washed with $H_2O$ and brine, dried (anhydrous $MgSO_4$), filtered, concentrated and flash chromatographed (silica gel, 50% ether in hexane). After concentration of appropriate fractions, 2.80 g (75%) of 64 as a yellow oil was obtained.

64 Methyl 2-(p-methoxy-a-toluenylthio)-3-nitrobenzoate:

1H NMR (90 MHz, $CDCl_3$) $\delta$=3.73, 3.90 (S, 3H each) $OCH_3$); 4.08 (S, 2H, $SCH_2$); 6.7-7.8 (m, 7H, ArH).

STEP 2: The methyl benzoate 64 was treated similarly to compound

44

(Example 7) with diisobutyl aluminum hydride at -78° C. The crude product 65 was isolated as a yellow oil in 96% yield.

65

2-(p-methoxy-a-toluenethio)-3-nitrobenzyl alcohol:
'H NMR (90 MHz, $CDCl_3$) $\delta$ = 2.20 (bt, J = 5.0 Hz, 1H, OH); 3.74 (S, 3H, $OCH_3$); 3.95 (S, 2H, $SCH_2$); 4.63 (bd, J = 5.0 Hz, 2H, $OCH_2$); 6.68-7.70 (m, 7H, ArH).

STEP 3: Treatment of alcohol

65

under conditions similar to alcohol

57

(Example 9) gave an 80% yield of

66

as a yellow solid from ether/hexane.

66

1-(p-methoxy-a-toluenethio)-2-nitrobenzaldehyde:
'H NMR (90 MHz, $CDCl_3$) $\delta$ = 3.80 (S, 3H, $OCH_3$); 4.00 (S, 2H, $SCH_2$); 6.70-7.10 (m, 4H, ArH); 7.50-8.10 (m, 3H, ArH); 10.20 (s, 1H, CHO). m.p. 95.0-97.5° C.

STEP 4: Dihydropyridine 67 was obtained from aldehyde

66

by the same conditions as used for dihydropyridine

59

(Example 9). 67 crystallized from ether/hexane as a yellow solid.

67

Diethyl 1,4-dihydro-4-(2-(((4-methoxyphenyl) methyl)thio)-3-nitrophenyl)-2,6-dimethyl-3,5-pyridinedicarboxylate:
'H NMR (360 MHz, $CDCl_3$) $\delta$ = 1.17 (t, J = 7.5 Hz, 6H, $CH_2CH_3$); 2.27 (S, 6H, $CH_3$); 3.80 (S, 3H, $OCH_3$); 4.07 (S, 2H, $SCH_2$); 4.08 (m, 4H, $OCH_2$); 5.59 (bs, 1H, NH); 5.81 (S, 1H, ArCH); 6.84 (d, J = 8.0 Hz, 2H, ArH); 7.33 (d, J = 8.0 Hz, 2H, ArH); 7.34 (m, zH, ArH); 7.62 (dd, J = 7.5, 1.5 Hz, 1H, ArH).

STEP 5: Dihydro pyridine 67 was reacted under conditions similar to dihydropyridine 52 (Example 8) to give the two benzothiazocine diastereomers

68

and 69 which were separated by flash chromatography (silica gel, 2.5% acetone in methylene chloride) and crystalized from ether/hexane.

68 'H NMR (360 MHz, $CDCl_3$) $\delta$ = 1.05, 1.31 (t, J = 7.5 Hz, 3H each, $CH_2CH_3$); 1.96 (S, 3H, $SCCH_3$); 2.24 (S, 3H, $CH_3$); 3.01 (d, J = 4.0 Hz, 1H, $HCCO_2$); 4.01 (q, J = 7.5 Hz, 2H, $OCH_2$); 4.07-4.25 (m, 2H, $OCH_2$); 4.77 (d, J = 4.0 Hz, 1H, ArCH); 4.80 (6S, 1H, NH); 7.11 (t, J = 7.5 Hz, 1H, ArH); 7.76 (dd, J = 7.5 Hz, 1.5 Hz, 1H, ArH); 8.07 (dd, J = 7.5, 1.5 Hz, 1H, ArH).

69 'H NMR (360 MHz, $CDCl_3$) $\delta$ = 1.27, 1.30 (t, J-7.5Hz, 3H each, $CH_2CH_3$); 1.95 (s, 3H, $SCCH_3$); 2.26 (s, 3H, $CH_3$); 2.92 (t, J-1.5Hz, 1H, $HCCO_2$); 4.05-4.25 (m, 4H, $OCH_2$); 4.68 (bs, 1H, ArCH); 4.68 (bs, 1H, NH); 7.14 (t, J = 9.0Hz, 1H, ArH); 7.82 (d, J = 9.0Hz, 1H, ArH);
8.07 (d, J = 9.0Hz, 1H, ArH).

EXAMPLE 11

Diethyl 3,6-dihydro-2,4-dimethyl-10-nitro-2,6-methano-2H-1,3-benzoxazocine-5,11-dicarboxylate (74&75)

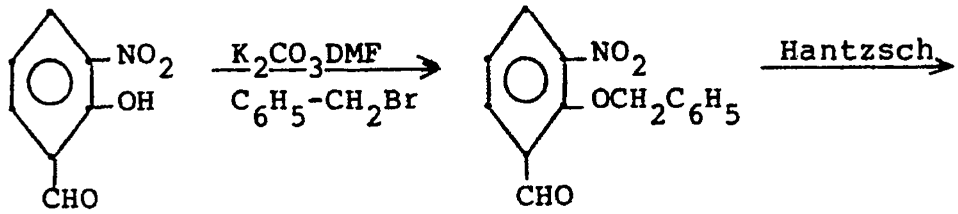


70 71

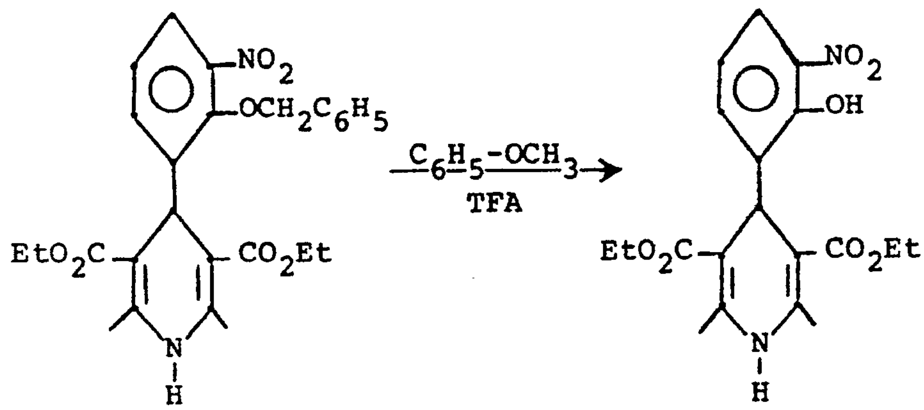


72 73

and

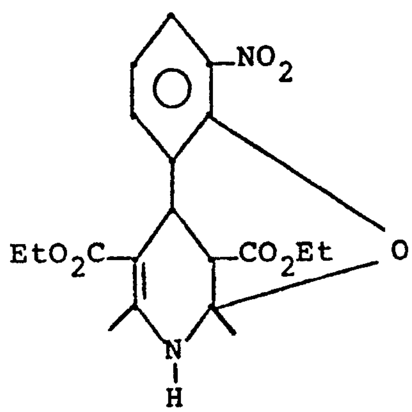

74 & 75

Step 1: A mixture of

70

(16.7 g, 0.1 mole), pulverized potassium carbonate (13.8 g, 0.1 mole), and benzyl bromide (48 ml, 0.4 mole) in DMF (100 ml) was stirred overnight at room temperature. $H_2O$ was added and the separated solid was filtered off and washed with $H_2O$, then with ether and dried to yield 16.2 g (63%) of

71.

Compound

71

was condensed under similar conditions to give the dihydropyridine

72.

71

'H NMR (90 MHz, $CDCl_3$): δ 5.2 (s, 2H, $-OCH_2$); 7.4 (s, 5H, $C_6H_5$); 7.4 (m, 1H, ArH); 8.0-8.2 (m, 2H, ArH), 10.25 (s, 1H, -CHO).

72

'H NMR (360 MHz, $CDCl_3$): δ 1.13 (t, J-7.5Hz, 6H, $-CH_2CH_3$); 2.17 (s, 6H, $-CH_3$); 3.95-4.08 (m, 4H, $-CH_2CH_3$); 4.97 (s, 2H, $-CH_2$); 5.2 (bs, 1H, -NH); 5.35 (s, 1H, ArCH); 7.08 (t, J=7.5Hz, 1H, ArH); 7.3-7.4 (m, 3H, ArH); 7.52 (bd, 2H, ArH); 7.58 (dd, J=1.5, 7.5Hz, 1H, ArH); 7.69 (dd, J=1.5, 7.5Hz, 1H, ArH). M.p. 147-149 °C.

Step 2: Anisole (5 ml) was degassed and then

72

(2 g) was added. The reaction mixture was protected from light and stirred under nitrogen and trifluoroacetic acid (5 ml) was added. The resulting yellow solution was stirred at room temperature for 15 minutes, a saturated solution of $NaHCO_3$ (5 ml) was added, and the mixture extracted with $CH_2Cl_2$. The extract was dried ($Na_2SO_4$), filtered and concentrated in vacuo to yield an oil which was a mixture of

73, 74 and 75.

Flash chromatography (silica gel, 1:1 ether-hexane) separated

73

from the mixture of

74 and 75.

A solution of

73

in $CHCl_3$ containing a catalytic amount of dl-10-camphorsulfonic acid was stirred at room temperature for 15 minutes and then concentrated in vacuo. The residue, which contained a mixture of

73, 74 and 75

was chromatographed, and the recovered

73

was again reacted. This procedure provided a sufficient quantity of

74

and

75

to be obtained for separation and purification. The mixture of

74 and 75

was flash chromatographed (silica gel, 1:1 ether-hexane) to yield

74

as a yellow solid (from ether) and

75'

as a yellow solid (from ether). An analytical sample of

73

was obtained from ether.

73

'H NMR (360 MHz, $CDCl_3$): $\delta$ 1.19 (t, J=7.5Hz, 6H, $-CH_2CH_3$); 2.32 (s, 6H, $-CH_3$); 4.0-4.15 (m, 4H, $-CH_2CH_3$); 5.35 (s, 1H, ArCH); 5.67 (bs, 1H, -NH); 6.85 (t, J=7.5Hz, 1H, ArH); 7.6 (dd, J=1.5, 9Hz, 1H, ArH); 7.95 (dd, J=1.5, 9Hz, 1H, ArH). m.p. 150-151 °C.

74

(Fast isomer) 'H NMR (360 MHz, $CDCl_3$) : $\delta$ 1.25, 1.35 (t, J=7.5Hz, 3H each, $-CH_2CH_3$); 1.98 (s, 3H, $-OCCH_3$); 2.23 (s, 3H, $-CH_3$); 2.97 (dd, J=3.0, 1.5Hz, 1H, $CHCO_2$); 4.07-4.26 (m, 4H, $-CH_2CH_3$); 4.7 (d, J=3.0Hz, 1H, ArCH); 5.13 (bs, 1H, -NH); 6.9 (t, J=7.5Hz, 1H, ArH); 7.6 (dd, J=1.5, 7.5Hz, 1H, ArH); 7.66 (dd, J=1.5, 7.5Hz, 1H, ArH). m.p. 130-139 °C.

75

(Slow isomer) 'H NMR (360 MHz, $CDCl_3$): δ 1.11, 1.35 (t, J=7.5Hz, 3H each, $-CH_2CH_3$); 1.9 (s, 3H, $OCCH_3$); 2.21 (s, 3H, $-CH_3$); 2.87 (d, J=3.0Hz, 1H, $CHCO_2$); 4.05-4.28 (m, 4H, $-CH_2CH_3$); 4.55 (d, J=3.0Hz, 1H, ArCH); 5.04 (bs, 1H, -NH); 6.9 (t, J=7.5Hz, 1H, ArH); 7.58 (dd, J=1.5, 7.5Hz, 1H, ArH); 7.66 (dd, J=1.5, 7.5Hz, 1H, ArH). m.p. 168-169 °C.

EXAMPLE 12

Diethyl 3,6-dihydro-2,4-dimethyl-9-nitro-2,6-methano-2H-1,3-benzoxazocine-5,11-dicarboxylate (77&78)

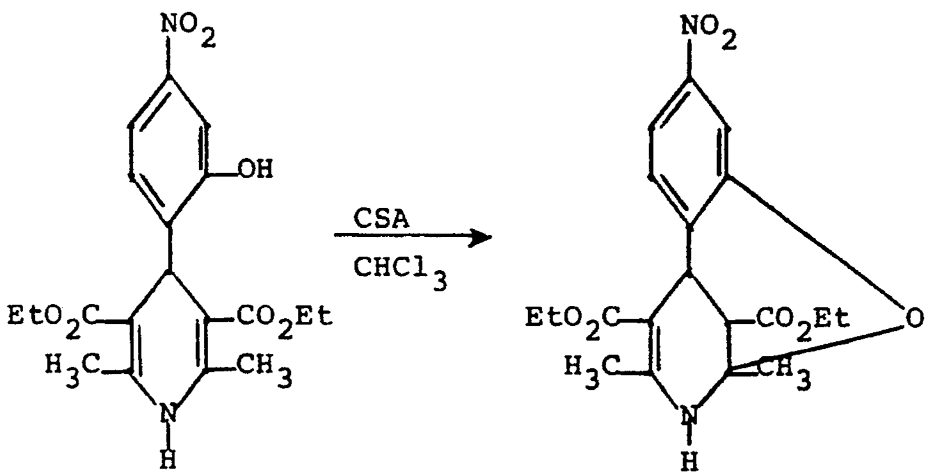


76      77 & 78

Phenol

76

was prepared in a similar fashion to phenol

17

(Example 1).

76

'H NMR (90 MHz, $CDCl_3$): δ 1.2 (t, J=7.5Hz, 6H, $-CH_2CH_3$); 2.35 (s, 6H, $-CH_3$); 4.1 (q, J=7.5Hz, 4H, $-CH_2CH_3$); 5.1 (s, 1H, ArCH); 6.2 (bs, 1H, NH); 7.1-7.7 (m, 3H, ArH); 9.4 (bs, 1H, -OH).

Benzoxazocines 77 and 78 were prepared simarlarly to

18

and

19

(Example 1).

77 Fast isomer 'H NMR (360 MHz, $CDCl_3$): δ 1.25, 1.35 (t, J=7.5Hz, 3H each, $-CH_2CH_3$); 1.93 (s, 3H, $OCCH_3$); 2.22 (s, 3H, $-CH_3$); 2.95 (dd, J=1.5, 3.0Hz, 1H, $CHCO_2$); 4.07-4.26 (m, 4H, $-CH_2CH_3$); 4.7 (d, J=3.0Hz, 1H, ArCH); 5.05 (bs, 1H, -NH), 7.5 (d, J=9.0Hz, 1H, ArH); 7.65 (d, J=3.0Hz, 1H, ArH), 7.7 (dd, J=3.0, 9.0Hz, 1H, ArH). m.p. 144-147° C.

78

Slow isomer 'H NMR (360 MHz, $CDCl_3$): δ 1.15, 1.35 (t, J=7.5Hz, 3H each, $-CH_2CH_3$); 1.87 (s, 3H, $OCCH_3$); 2.2 (s, 3H, $CH_3$); 2.87 (d, J=2Hz, 1H, $CHCO_2$); 3.85-4.1 (m, 4H, $-CH_2CH_3$); 4.37 (d, J=2Hz, 1H, ArCH); 4.75 (bs, 1H, NH); 7.5 (d, J=8Hz, 1H, ArH); 7.65 (d, J=2Hz, 1H, ArH); 7.73 (dd, J=2.0, 8.0Hz, 1H, ArH). m.p. broad, clear melt at 115° C.

EXAMPLE 13

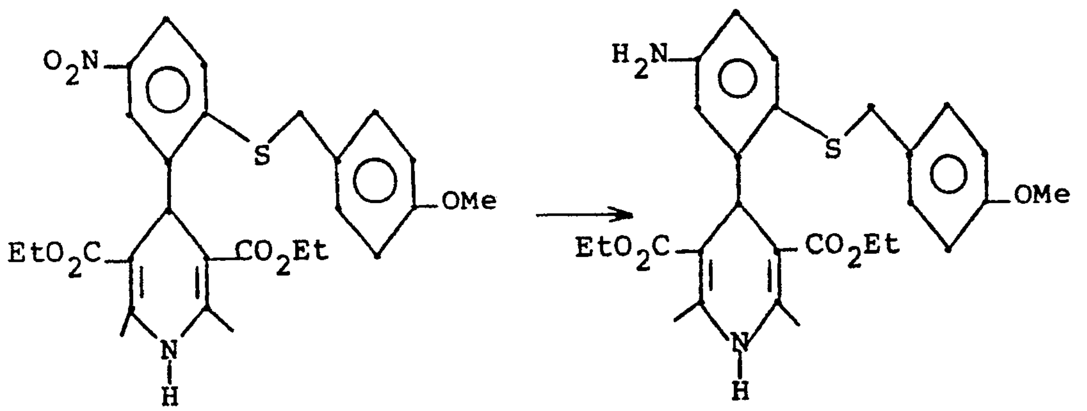

79 80

0.526 Grams of dihydropyridine

79

in 3 mL of anhydrous tetrahydrofuran can be treated with 150 mg of lithium aluminum hydride at 0° C. This can then be warmed to 25° C and the amine isolated in the usual manner to give 300 mg of

80.

EXAMPLE 14

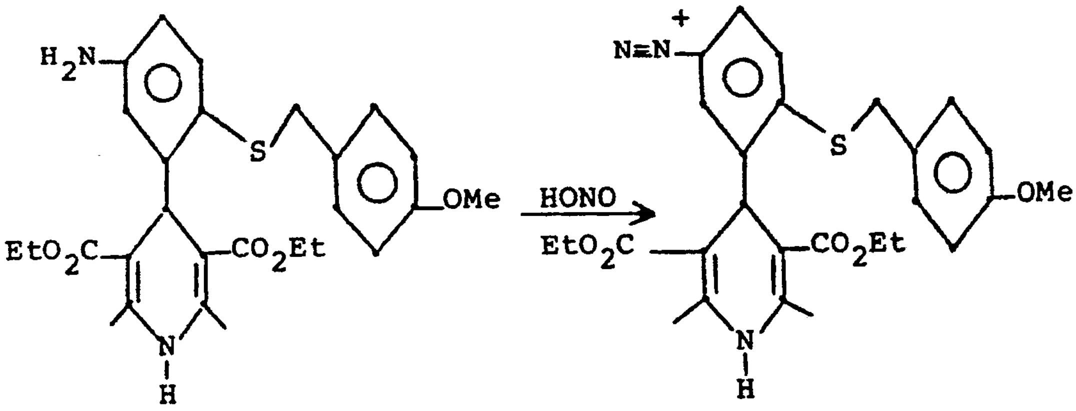


80 81

0.500 Grams of amine

80

can be treated using the general procedure with nitrous acid to generate the diazonium salt

81

and this salt can be used for further transformation.

EXAMPLE 15

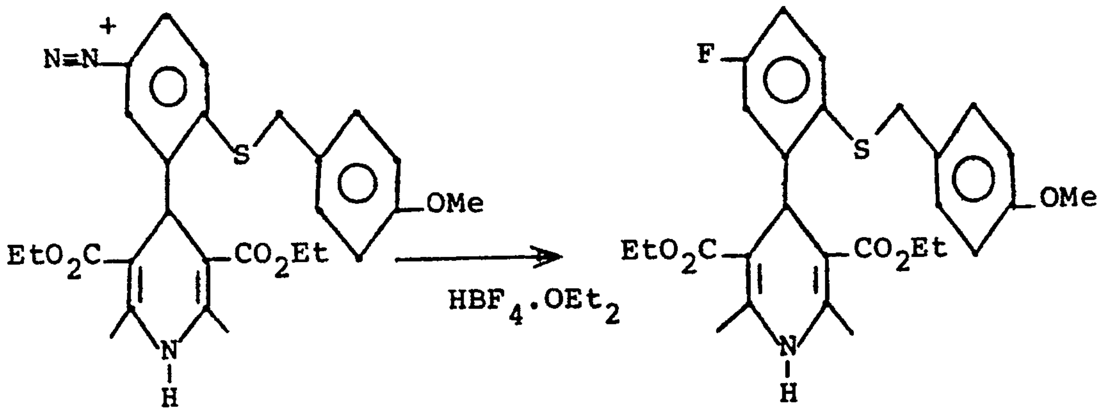


81 82

0.40 Grams of diazonium salt

81

can be treated with 0.33 g of tetrafluoroboric acid diethyl ether complex and the precipitate collected and heated to give, after the usual isolation, fluorodihydropyridine 82 (0.15 g).

EXAMPLE 16

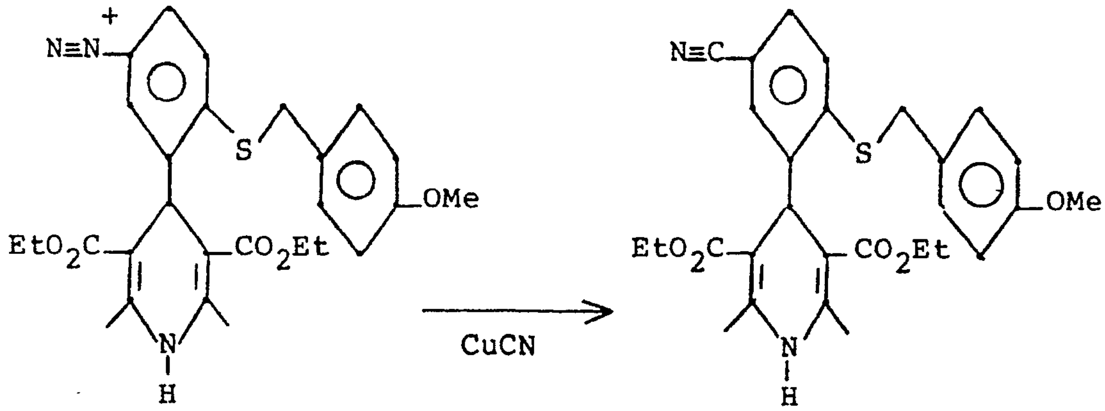


81 83

0.30 Grams of diazonium salt

81

can be treated with 0.50 g of copper (I) cyanide to give, after the usual workup, nitrile

83

(0.20 g).

EXAMPLE 17

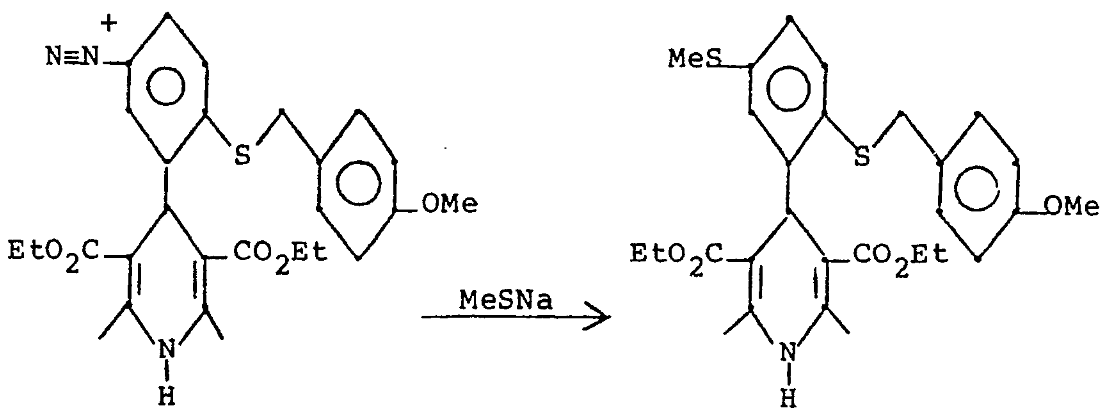


81 84

0.30 Grams of diazonium salt

81

can be treated with sodium methylmercaptide (.2 g) which, when treated under usual conditions, will yield the methylthio dihydropyridine

84

(0.10 g).

EXAMPLE 18

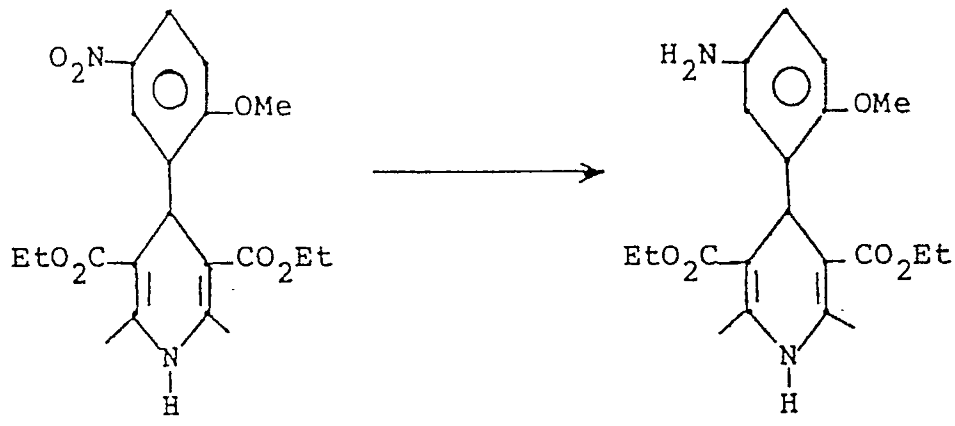

15 85

1.00 Gram of compound

15

(Example 1) can be treated with 5% Pd on charcoal under an atmosphere of hydrogen in ethanol to produce dihydropyridine

85

(0.90 g) which can be isolated in the usual manner.

EXAMPLE 19

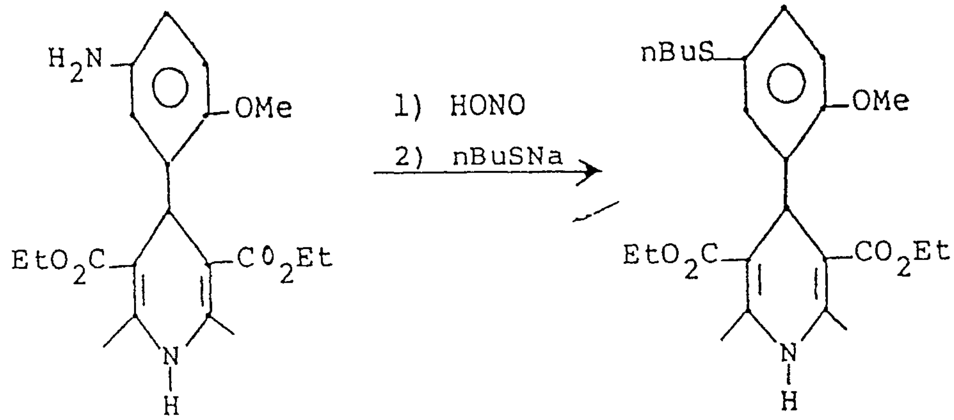


Dihydropyridine

85

(0.50 g) can be diazotized in a manner similar to

17

(Example 1) to give the diazonium species which can then be treated with sodium n-butylmercaptide to give dihydropyridine

86.

EXAMPLE 20

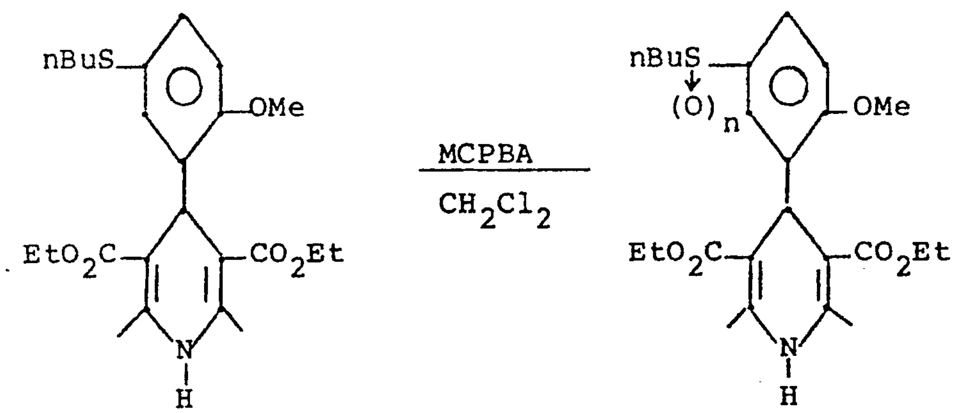


86

87 (n=1)

88 (n=2)

Dihydropyridine

86

can be oxidized with metachloroperbenzoic acid in $CH_2Cl_2$ to give mixtures of sulfoxide

87

and sulfone

88.

Compounds

87

and

88

can then be separated from each other by chromatography on silica gel.

EXAMPLE 21

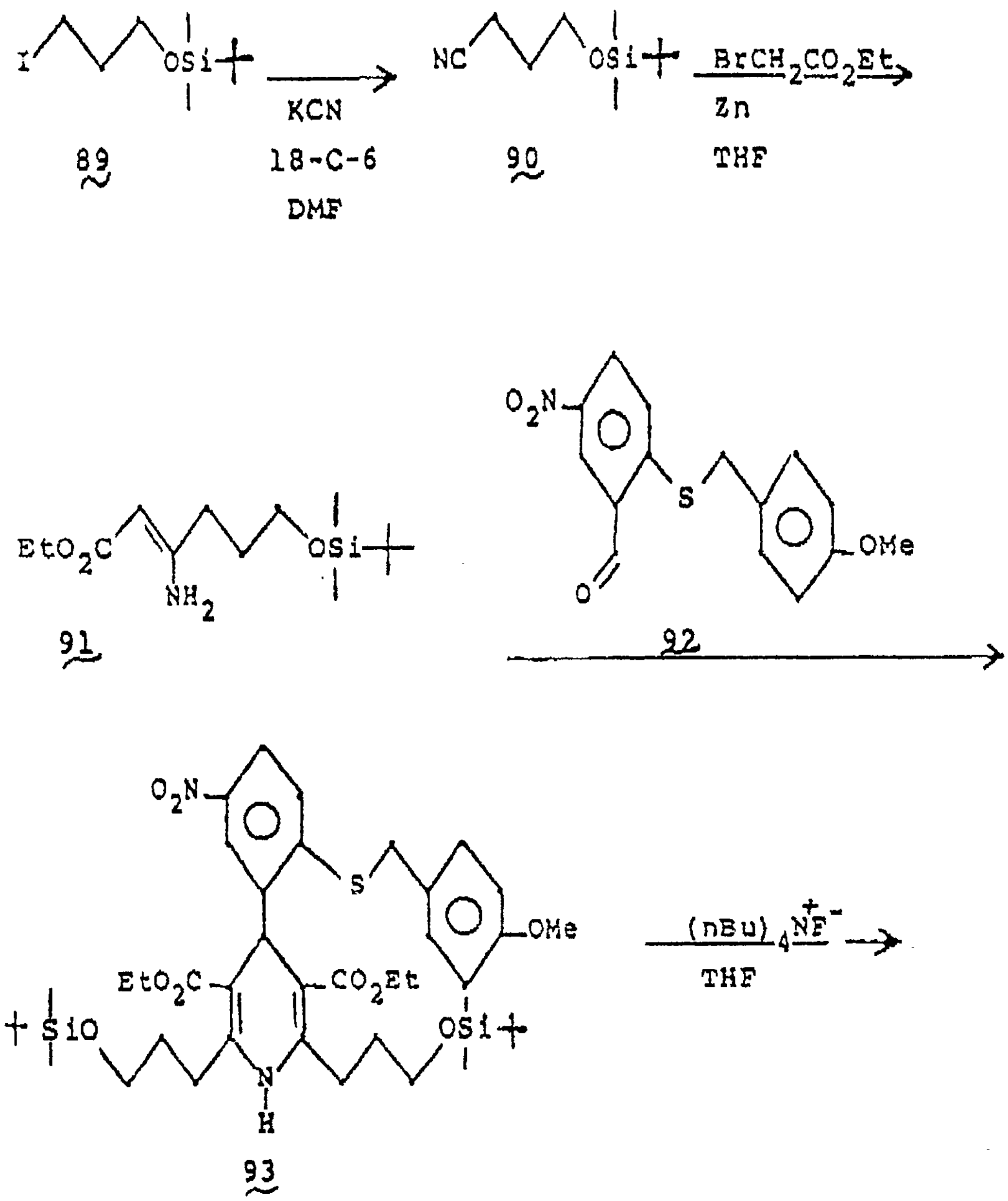


89 90 91 92 93

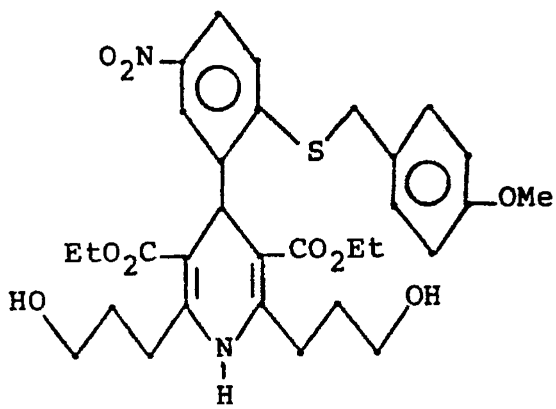

94

The known iodide

89

(10.0 g) can be treated with 10 g of potassium cyanide in 100 mL of DMF followed by 0.87 g of 18-crown-6 for 20 hours and extracted into ether. After usual isolation procedures, the nitrile

90

can be isolated (6.0 g). Nitrile

90

(6.0 g) can be treated with 9.40 g of zinc dust and 12.6 mL of ethyl bromo acetate in 130 mL of tetrahydrofuran for one hour at reflux to give, after usual workup, amino-crotonate

91

(8.00 g). Compound

91

(8.0 g) can be refluxed with 4.40 g of

92

in isopropyl alcohol for 12 hours to give dihydropyridine

93

(3.80 g).

Dihydropyridine

93

(3.0 g) can be treated with 10 mL of 1.0 molar (nBu)$_4$NF in tetrahydrofuran at 25° C for 1.5 hours to give, after isolation, diol

94.

EXAMPLE 22

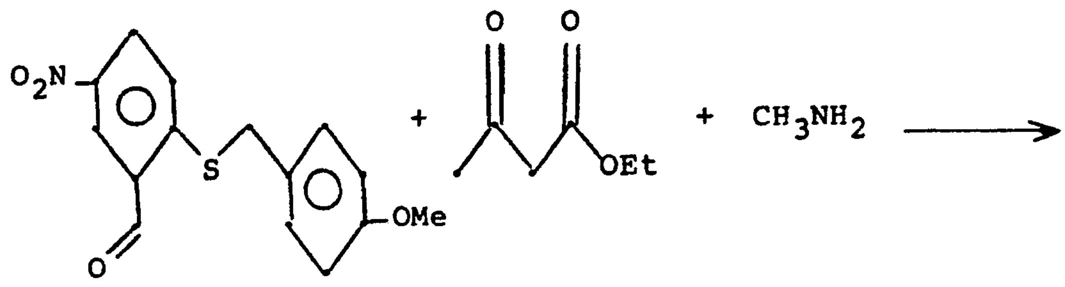

95

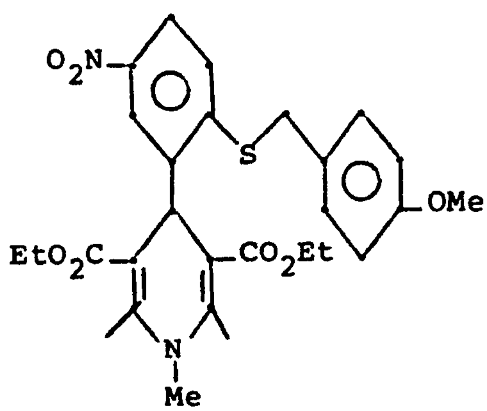

96

Aldehyde

95

(3.0 g), 3.0 g of ethyl acetate, and 10 mL of 40% methylamine in $H_2O$ can be refluxed in 10 mL of isopropanol for 12 hours. After usual isolation procedure, dihydropyridine

96

can be obtained (1.50 g).

EXAMPLE 23

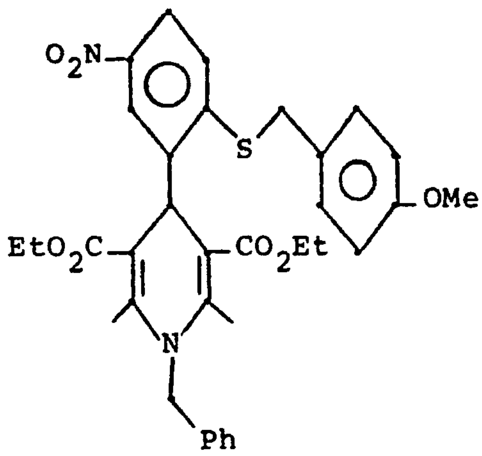

97

Following the procedure of Example 22, benzyl amine can be used to obtain N-benzyldihydropyridine

97.

EXAMPLE 24

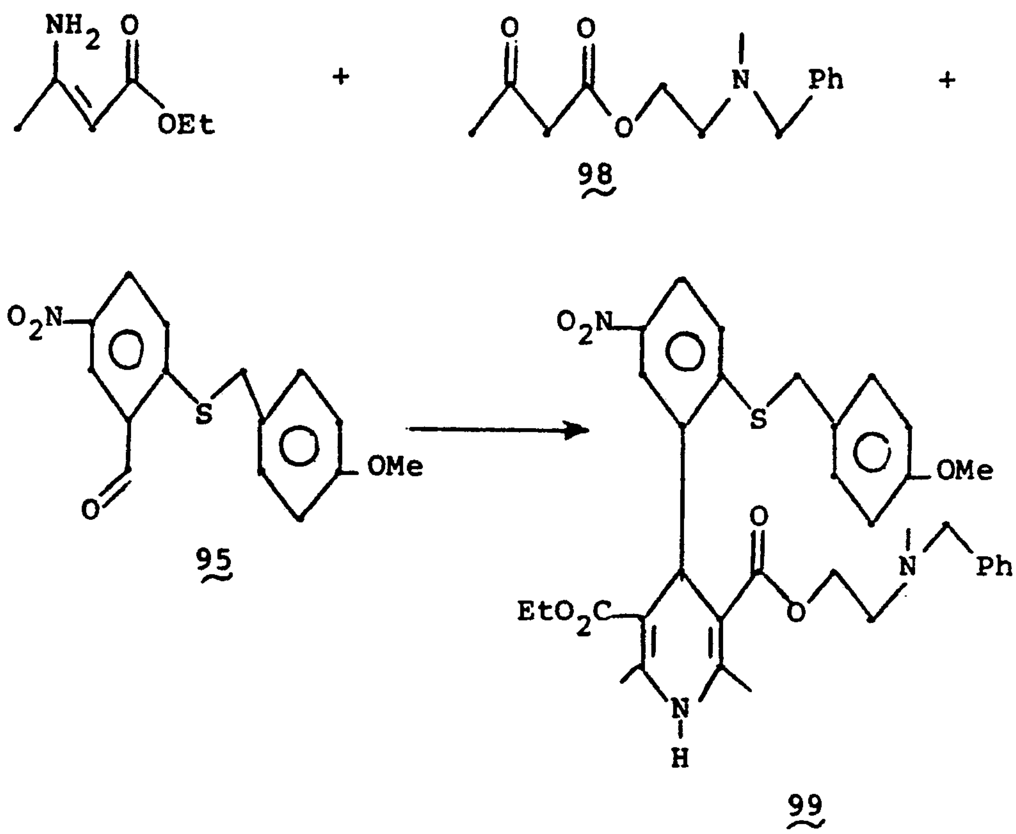

99

Dihydropyridine

99

can be prepared from the acetoacetate

98

using conditions outlined in the literature (Chem. Pharm. Bull. 27 (6) 1426-1440 (1979)).

EXAMPLE 25

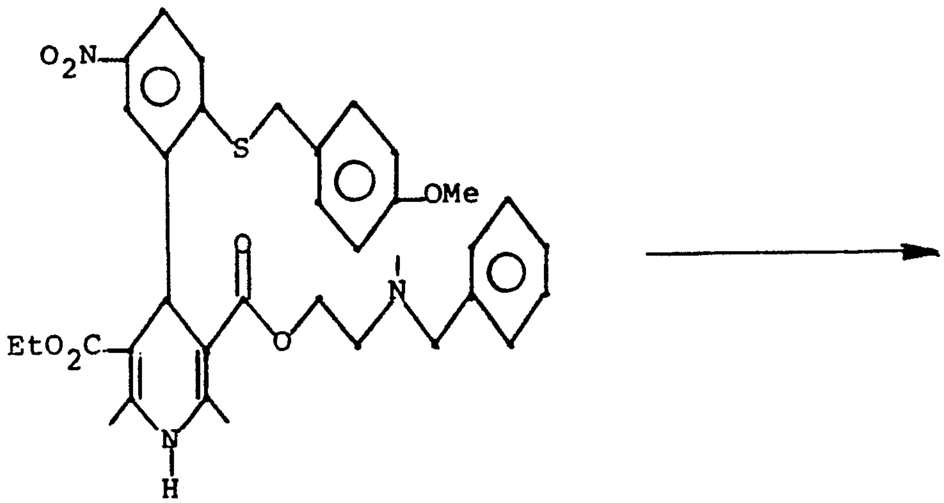

99

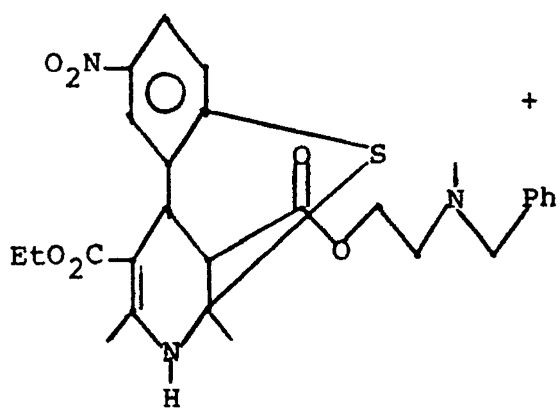

+

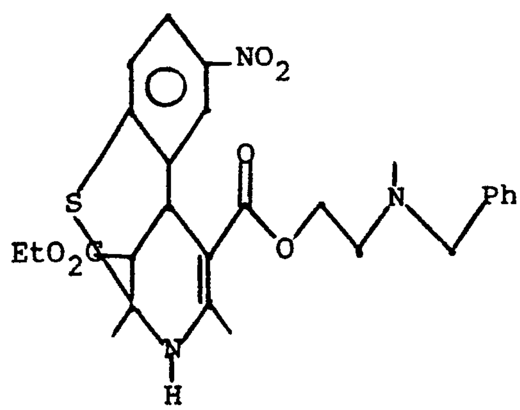

100 101

Dihydropyridine

99

can be used to prepare both dl pairs of diastereomers

100 and 101

by treatment with trifluoroacetic acid in anisole.

Following the methods shown and described in the Reaction Schemes and using the procedures set forth in the Examples, additional compounds of Formula 1 can be prepared as set forth in Table I below:

TABLE I

Additional Compounds of Formula 1

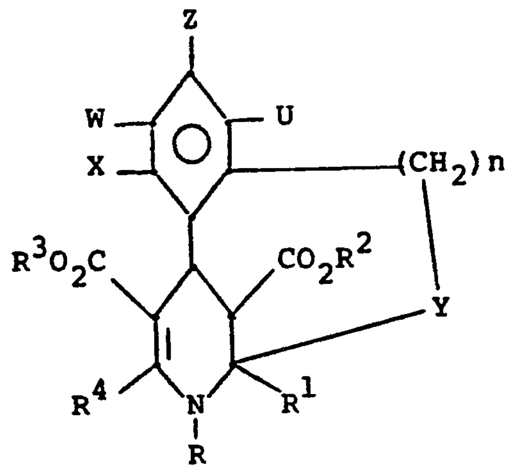

1

| Compound | X | W | Z | U | n | Y | Method |
|---|---|---|---|---|---|---|---|
| (i) | $CH_3$ | H | H | H | 0 | O | A, B or C |
| (ii) | H | -CN | do | do | do | S | F |
| (iii) | do | $NH_2$ | do | do | do | do | F |
| (iv) | do | F | do | do | do | do | F |
| (v) | do | $CH_3S$ | do | do | do | do | F |
| (vi) | do | $NH_2$ | do | do | do | O | B |
| (vii) | do | n-butyl-S | do | do | do | do | C |
| (viii) | do | n-butyl-$\overset{\overset{O}{\|}}{S}$ | do | do | do | do | C |
| (ix) | do | n-butyl$SO_2$ | do | do | do | do | C |
| (x) | do | $NO_2$ | do | do | do | S | F |
| (xi) | do | do | do | do | do | do | F |
| (xii) | do | do | do | do | do | do | F |
| (xiii) | benzyl | do | do | do | do | do | F |
| (xiv) | H | H | do | do | do | O | C |
| (xv) | do | do | do | $CH_2C{\equiv}CCH_3$ | do | do | C |
| (xvi) | $CH_3$ | do | do | $CH_2{=}CHCH_2$ | do | do | A |
| (xvii) | H | $CH_3\overset{\overset{O}{\|}}{C}NH$ | do | H | 1 | S | F |
| (xviii) | do | $NO_2$ | do | do | 0 | do | F |
| (xix) | do | do | do | do | do | do | F |
| (xx) | do | do | do | do | 1 | do | F |
| (xxi) | do | do | do | $NO_2$ | 0 | do | F |
| (xxii) | do | H | do | do | 1 | do | F |
| (xxiii) | do | do | $NO_2$ | H | 0 | do | F |
| (xxiv) | do | do | do | do | do | O | A, C or D |
| (xxv) | do | do | H | Ph | do | S | F |
| (xxvi) | do | do | do | $NO_2$ | do | do | F |
| (xxvii) | do | $NO_2$ | do | H | do | O | A, C or D |

| Compound | X | W | Z | U | n | Y | Method |
|---|---|---|---|---|---|---|---|
| (xxviii) | do | do | do | do | do | S | F |
| (xxix) | do | do | do | do | do | do | F |
| (xxx) | do | do | do | do | do | do | F |

| Compound | R | $R^1$ | $R_4$ | $R^2$ | $R^3$ |
|---|---|---|---|---|---|
| (i) | H | ethyl | ethyl | isopropyl | isopropyl |
| (ii) | do | $CH_3$ | $CH_3$ | ethyl | ethyl |
| (iii) | do | do | do | do | do |
| (iv) | do | do | do | do | do |
| (v) | do | do | do | do | do |
| (vi) | do | do | do | do | do |
| (vii) | do | do | do | do | do |
| (viii) | do | do | do | do | do |
| (ix) | do | do | do | do | do |
| (x) | do | $HO(CH_2)_3$ | $HO(CH_2)_3$ | do | do |
| (xi) | do | do | $CH_3$ | do | do |
| (xii) | do | $CH_3$ | $HO(CH_2)_3$ | do | do |
| (xiii) | do | do | $CH_3$ | do | do |
| (xiv) | do | do | do | 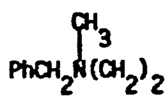 | CH3 |
| (xv) | do | do | do | $CH_3$ | 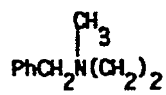 |
| (xvi) | do | ethyl | ethyl | do | $CH_3$ |
| (xvii) | do | do | $CH_3$ | ethyl | ethyl |
| (xviii) | do | do | do | cyclohexyl | do |
| (xix) | do | do | do | ethyl | cyclohexyl |
| (xx) | do | isopropyl | isopropyl | do | ethyl |
| (xxi) | do | ethyl | cyclohexyl | do | do |
| (xxii) | do | $CH_3$ | $CH_2$-cyclohexyl | $CH_3$ | $CH_3$ |
| (xxiii) | benzyl | do | $CH_3$ | do | do |
| (xxiv) | $CH_3$ | do | do | ethyl | ethyl |
| (xxv) | isopropyl | do | do | do | do |
| (xxvi) | H | ethyl | ethyl | cyclopropyl | $CH_3$ |
| (xxvii) | do | $CH_3$ | $CH_3$ | $CH_3$ | cyclopropyl |
| (xxviii) | do | do | do | $C_3H_7O(CH_2)_2$ | $C_3H_7O(CH_2)_2$ |

| Compound | R | $R^1$ | $R_4$ | $R_2$ | $R_3$ |
|---|---|---|---|---|---|
| (xxix) | do | do | do | $CH_3O(CH_2)O(CH_2)_2$ | $CH_3O(CH_2)_2O(CH_2)_2$ |
| (xxx) | do | do | do | 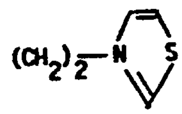 | 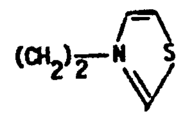 |

## Claims

1. A compound having the formula

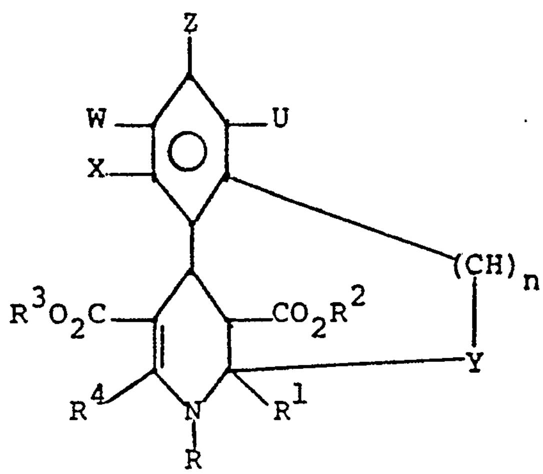

1

wherein:
R is hydrogen;
$C_1$-$C_8$ straight chain or branched alkyl;
benzyl;
$R^1$ and $R^4$ are independently
hydrogen;
straight chain or branched, saturated hydrocarbon having up to 8 carbon atoms;
hydroxy-$C_1$-$C_8$-alkyl;
$C_3$-$C_8$ cycloalkyl;
$CO_2$-loweralkyl of $C_1$-$C_8$
$R^2$ and $R^3$ are independently
straight chain or branched, saturated or unsaturated hydrocarbon having up to 8 carbon atoms;
$C_3$-$C_8$ cycloalkyl;
$C_2$-$C_8$ alkyl interrupted by 1 to 2 oxygen atoms or substituted with $NR^5R^6$ wherein $R^5$ and $R^6$ can independently be hydrogen, $C_1$-$C_8$ alkyl, aralkyl wherein the alkyl group has up to 8 carbon atoms and the aryl has 6 carbon atoms, or $R^5$ and $R^6$ together with the N atom can be joined to form a 5- or 6-membered ring wherein the 6-membered ring can contain an O or N heteroatom and the N heteroatom can be substituted with $C_1$-$C_6$ alkyl;;
X, W, Z and U can independently be:
hydrogen;
phenyl and substituted phenyl wherein the substituents can be 1-2 halo atoms;
halo;
$NO_2$;
trifluoromethyl;
$C_1$-$C_8$ alkoxy;

$C_1$-$C_8$ linear or branched alkyl, linear or branched alkenyl or alkynyl having up to 8 carbon atoms;
$C_1$-$C_8$ thioalkyl wherein the S atom can be substituted with 1-2 O atoms;
cyano;
$NH_2$;

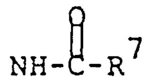

wherein $R^7$ is $C_1$-$C_8$ alkoxy or $C_1$-$C_8$ alkyl; provided that two of X, W, Z and U are hydrogen;
or, X and W, W and Z, or Z and U, together with the phenyl ring form naphthyl or benzooxadiazole rings;
Y is O; S; SO;
n is 0 or 1; and,
the pharmaceutically acceptable acid addition salts thereof; with the proviso that X cannot be $C_1$-$C_8$ alkoxy or $C_2$-$C_8$ alkenyl.

**2.** The compound of claim 1 wherein:
R is hydrogen;
$R^1$ and $R^4$ are independently $C_{1-8}$ alkyl;
$R^2$ and $R^3$ are independently $C_{1-8}$ alkyl which can be substituted with $NR^5R^6$ wherein $R^5$ and $R^6$ are as defined in Claim 1;
X is hydrogen or halo;
U and W can each be $NO_2$; $CF_3$; or halo;
Z is hydrogen;
Y is O; S; and,
n is 0 or 1.

**3.** The compound of claim 1 wherein:
R is hydrogen;
$R^1$ and $R^4$ are methyl
$R^2$ and $R^3$ are $C_1$-$C_4$ alkyl;
X is hydrogen or halo;
U and W can each be $NO_2$; $CF_3$; or halo;
Z is hydrogen;
Y is S; and,
n is 0 or 1.

**4.** A compound having the formula:

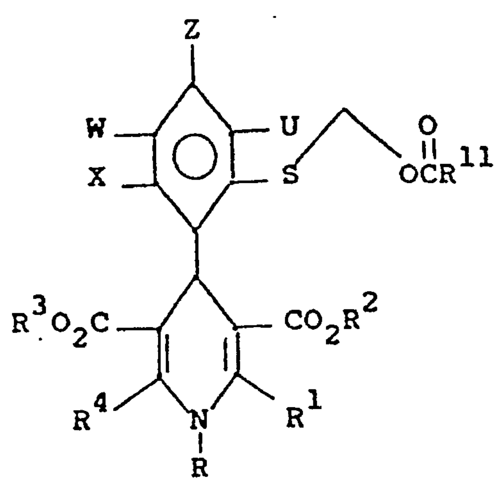

14

wherein R-$R^4$, X, W, Z and U are as defined in Claim 1; and, $R^{11}$ is $C_1$-$C_8$ loweralkyl.

**5.** A pharmaceutical composition useful for providing a cardiotonic effect, a vasodilating effect, an antiarrhythmic action on cardiac muscle, vascular spasmolytic action, antihypertensive effect, spasmolytic action on the smooth muscle of the gastrointestinal and urogenital tracts and the respiratory system, cerebral and peripheral circulation and protecting ischemic myocardium which composition consists essentially of a pharmaceutically acceptable carrier and a pharmaceutically effective amount of a compound of Claim 1.

**6.** The composition of Claim 5 wherein said pharmaceutical composition includes an additional pharmaceutical agent selected from the group, an angiotensin converting enzyme inhibitor, an antihypertensive, a diuretic, a $\beta$-blocker, and a cardiotonic agent as well as admixtures and combinations thereof.

**7.** The composition of Claim 6 wherein said additional pharmaceutical agent is selected from the group: enalapril, hydralazine hydrochloride, hydrochlorothiazide, methyldopa, timolol, and digitalis.

## Revendications

**1.** Composé répondant à la formule

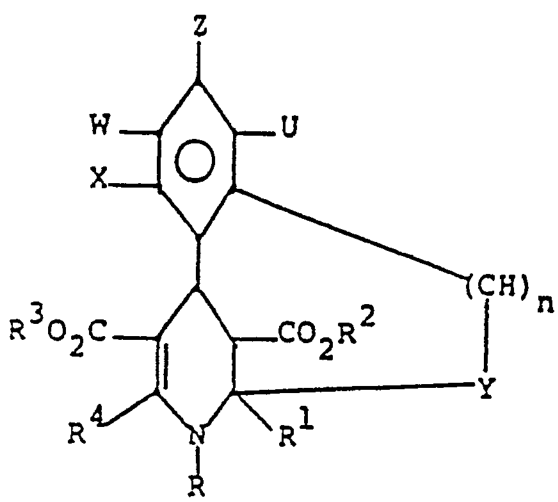

1

dans laquelle :
R est un hydrogène ;
un alkyle en $C_1$-$C_8$ à chaîne droite ou ramifiée ;
un benzyle ;
$R^1$ et $R^4$ sont indépendamment un hydrogène ;
un hydrocarbure saturé à chaîne droite ou ramifiée ayant jusqu'à 8 atomes de carbone ;
un hydroxyalkyle en $C_1$-$C_8$ ;
un cycloalkyle en $C_3$-$C_8$ ;
un $CO_2$-alkyle inférieur en $C_1$-$C_8$ ;
$R^2$ et $R^3$ sont indépendamment un hydrocarbure saturé ou insaturé à chaîne droite ou ramifiée ayant jusqu'à 8 atomes de carbone ;
un cycloalkyle en $C_3$-$C_8$ ;
un alkyle en $C_2$-$C_8$ interrompu par 1 ou 2 atomes d'oxygène ou substitué par $NR^5R^6$ où $R^5$ et $R^6$ peuvent indépendamment être un hydrogène, un alkyle en $C_1$-$C_8$ ou un aralkyle dont le groupe alkyle a jusqu'à 8 atomes de carbone et le groupe aryle a 6 atomes de carbone, ou $R^5$ et $R^6$ avec l'atome d'azote peuvent être unis pour former un cycle à 5 ou 6 chaînons où le cycle à 6 chaînons peut contenir un hétéroatome de O ou N et l'hétéroatome N peut être substitué par un alkyle en $C_1$-$C_6$ ;
X, W, Z et U peuvent indépendamment être :

un hydrogène ;
un phényle ou un phényle substitué dont les substituants peuvent être 1 ou 2 atomes d'halogène ;
un halogéno ;
$NO_2$ ;
un trifluorométhyle ;
un alcoxy en $C_1$-$C_8$ ;
un alkyle linéaire ou ramifié en $C_1$-$C_8$, un alcényle ou alcynyle linéaire ou ramifié ayant jusqu'à 8 atomes de carbone ;
un thioalkyle en $C_1$-$C_8$ dans lequel l'atome de S peut être substitué par 1 ou 2 atomes de O ;
un cyano ;
$NH_2$ ;

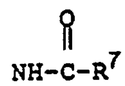

où $R^7$ est un alcoxy en $C_1$-$C_8$ ou un alkyle en $C_1$-$C_8$ ;
sous réserve que deux de X, W, Z et U soient un hydrogène ;
ou X et W, W et Z ou Z et U, avec le cycle phényle, forment des cycles naphtyle ou benzoxadiazole ;
Y est O ; S ; SO ;
n est 0 ou 1 ;
et leurs sels d'addition d'acides pharmaceutiquement acceptables ;
sous réserve que X ne peut pas être un alcoxy en $C_1$-$C_8$ ou un alcényle en $C_2$-$C_8$.

**2.** Composé selon la revendication 1 où :
R est un hydrogène ;
$R^1$ et $R^4$ sont indépendamment un alkyle en $C_1$-$C_8$ ;
$R^2$ et $R^3$ sont indépendamment un alkyle en $C_1$-$C_8$ qui peut être substitué par $NR^5R^6$ où $R^5$ et $R^6$ sont comme défini dans la revendication 1 ;X est un hydrogène ou un halogéno ;
U et W peuvent être chacun $NO_2$ ; $CF_3$ ; ou un halogéno ;
Z est un hydrogène ;
Y est O ou S ; et
n est 0 ou 1.

**3.** Composé selon la revendication 1 où :
R est un hydrogène ;
$R^1$ et $R^4$ sont un méthyle ;
$R^2$ et $R^3$ sont un alkyle en $C_1$-$C_4$ ;
X est un hydrogène ou un halogéno ;
U et W peuvent être chacun $NO_2$, $CF_3$ ou un halogéno ;
Z est un hydrogène ;
Y est S ; et
n est 0 ou 1.

**4.** Composé répondant à la formule :

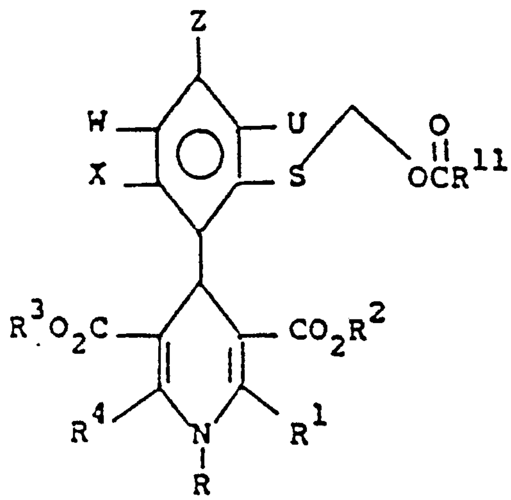

14

dans laquelle R-$R^4$, X, W, Z et U sont comme défini dans la revendication 1 ; et $R^{11}$ est un alkyle inférieur en $C_1$-$C_8$.

5. Composition pharmaceutique utile pour assurer un effet cardiotonique, un effet vaso-dilatateur, une action antiarythmique sur le muscle cardiaque, une action spasmolytique vasculaire, un effet antihypertensif, une action spasmolytique sur les muscles lisses des voies gastro-intestinales et génito-urinaires et du système respiratoire, la circulation cérébrale et périphérique et la protection du myocarde ischémique, cette composition étant constituée essentiellement d'un véhicule pharmaceutiquement acceptable et d'une quantité pharmaceutique efficace d'un composé selon la revendication 1.

6. Composition selon la revendication 5 qui comprend un agent pharmaceutique additionnel choisi parmi un inhibiteur de l'enzyme de conversion de l'angiotensine, un antihypertenseur, un diurétique, un β-bloquant et un agent cardiotonique, ainsi que leurs mélanges et associations.

7. Composition selon la revendication 6 dans laquelle ledit agent pharmaceutique additionnel est choisi parmi l'énalapril, le chlorhydrate d'hydralazine, l'hydrochlorothiazide, la méthyldopa, le timolol et les digitaliques.

**Patentansprüche**

1. Eine Verbindung, die die Formel

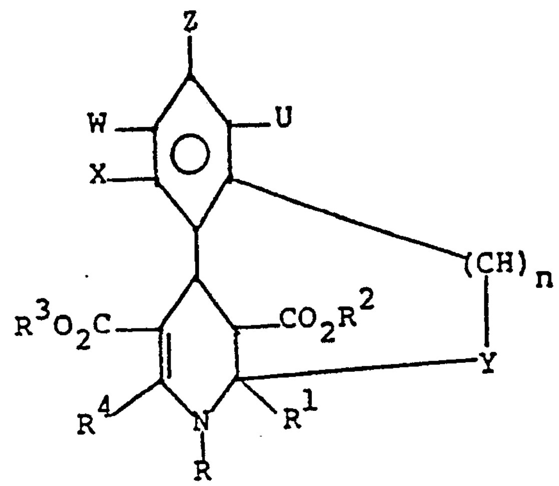

1

hat, worin:
R Wasserstoff,
geradkettiges oder verzweigtes $C_1$-$C_8$-Alkyl,
Benzyl ist,
$R^1$ und $R^4$ unabhängig
Wasserstoff,
geradkettiger oder verzweigter, gesättigter Kohlenwasserstoff mit bis zu 8 Kohlenstoffatomen,
Hydroxy-$C_1$-$C_8$-alkyl,
$C_3$-$C_8$-Cycloalkyl,
$CO_2$-Niederalkyl mit $C_1$-$C_8$ sind
$R^2$ und $R^3$ unabhängig
geradkettiger oder verzweigter, gesättigter oder ungesättigter Kohlenwasserstoff mit bis zu 8 Kohlenstoffatomen;
$C_3$-$C_8$-Cycloalkyl;
$C_2$-$C_8$-Alkyl sind, unterbrochen durch 1 bis 2 Sauerstoffatome oder substituiert mit $NR^5R^6$, worin $R^5$ und $R^6$ unabhängig Wasserstoff, $C_1$-$C_8$-Alkyl, Aralkyl, worin die Alkylgruppe bis zu 8 Kohlenstoffatome und Aryl 6 Kohlenstoffatome hat, sein können oder worin $R^5$ und $R^6$ zusammen mit dem N-Atom unter Bildung eines 5- oder 6-gliedrigen Ringes verbunden sein können, worin der 6-gliedrige Ring ein O- oder N-Heteroatom enthalten kann und worin das N-Heteroatom mit $C_1$-$C_6$-Alkyl substituiert sein kann,
X, W, Z und U unabhängig sein können:
Wasserstoff,
Phenyl und substituiertes Phenyl, worin die Substituenten 1 bis 2 Halogenatome sein können,
Halogen,
$NO_2$,
Trifluormethyl,
$C_1$-$C_8$-Alkoxy,
$C_1$-$C_8$- lineares oder verzweigtes Alkyl, lineares oder verzweigtes Alkenyl oder Alkinyl mit bis zu 8 Kohlenstoffatomen,
$C_1$-$C_8$-Thioalkyl, worin das S-Atom mit 1 bis 2 O-Atomen substituiert sein kann,
Cyan,
$NH_2$,

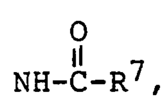

worin $R^7$ $C_1$-$C_8$-Alkoxy oder $C_1$-$C_8$-Alkyl ist, vorausgesetzt, dass zwei der Reste X, W, Z und U Wasserstoff sind,

oder X und W, W und Z oder Z und U, zusammen mit dem Phenylring Naphthyl- oder Benzooxadiazolringe bilden,
Y O, S, SO ist,
n 0 oder 1 ist, und,
die pharmazeutisch annehmbaren Säureadditionssalze davon, unter der Voraussetzung, dass X nicht $C_1$-$C_8$-Alkoxy oder $C_2$-$C_8$-Alkenyl sein kann.

**2.** Die Verbindung nach Anspruch 1, worin
R Wasserstoff ist,
$R^1$ und $R^4$ unabhängig $C_{1-8}$-Alkyl sind,
$R^2$ und $R^3$ unabhängig $C_{1-8}$-Alkyl sind, das mit $NR^5R^6$ substituiert sein kann, worin $R^5$ und $R^6$ wie im Anspruch 1 definiert sind,
X Wasserstoff oder Halogen ist,
U und W jeweils $NO_2$, $CF_3$ oder Halogen sein können,
Z Wasserstoff ist,
Y O, S ist und
n 0 oder 1 ist.

**3.** Die Verbindung nach Anspruch 1, worin
R Wasserstoff ist,
$R^1$ und $R^4$ Methyl sind,
$R^2$ und $R^3$ $C_1$-$C_4$-Alkyl sind,
X Wasserstoff oder Halogen ist,
U und W jeweils $NO_2$, $CF_3$ oder Halogen sein können,
Z Wasserstoff ist,
Y S ist und
n 0 oder 1 ist.

**4.** Eine Verbindung, die die Formel:

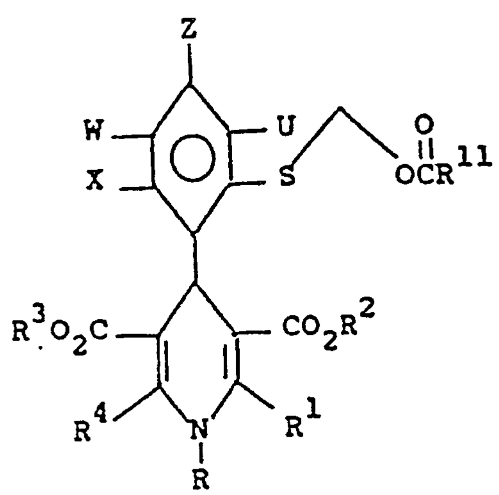

14

hat, worin R-$R^4$, X, W, Z und U wie im Anspruch 1 definiert sind und worin $R^{11}$ $C_1$-$C_8$-Niederalkyl ist.

**5.** Eine pharmazeutische Zusammensetzung, brauchbar zur Herbeiführung einer cardiotonischen Wirkung, einer vasodilatatorischen Wirkung, einer antiarrhythmischen Wirkung auf den Herzmuskel, einer vasculären, spasmolytischen Wirkung, einer blutdrucksenkenden Wirkung, einer spasmolytischen Wirkung auf den glatten Muskel des gastrointestinalen und urogenitalen Traktes und des Atmungssystems, für die cerebrale und peripherische Zirkulation und für den Schutz des ischämischen Herzmuskels, wobei die Zusammensetzung im wesentlichen aus einem pharmazeutisch annehmbaren Träger und einer

pharmazeutisch wirksamen Menge einer Verbindung nach Anspruch 1 besteht.

6. Die Zusammensetzung nach Anspruch 5, worin die pharmazeutische Zusammensetzung einen zusätzlichen pharmazeutischen Wirkstoff enthält, ausgewählt aus der Gruppe Inhibitor für Angiotensin umwandelndes Enzym, blutdrucksenkendes Mittel, Diuretikum, $\beta$-Blocker und cardiotonisches Mittel, ebenso wie Mischungen und Kombinationen davon.

7. Die Zusammensetzung nach Anspruch 6, worin der zusätzliche pharmazeutische Wirkstoff ausgewählt ist aus der Gruppe: Enalapril, Hydralazin-Hydrochlorid, Hydrochlorothiazid, Methyldopa, Timolol und Digitalis.